# EUROPEAN PATENT SPECIFICATION

(11) **EP 2 475 301 B1**
(45) Date of publication and mention of the grant of the patent: **10.07.2019**
(21) Application number: 10740671.2
(22) Date of filing: 10.08.2010
(51) Int. Cl.: A61B 34/10, A61B 5/103, A61B 90/00

(54) **DETERMINING A PLANE OF AN ANATOMICAL BODY PART**
BESTIMMUNG EINER FLÄCHE EINES ANATOMISCHEN KÖRPERTEILS
DÉTERMINATION D'UN PLAN D'UNE PARTIE DE CORPS ANATOMIQUE

(30) Priority: 08.09.2009 WO PCT/EP2009/061623
(43) Date of publication of application: 18.07.2012
(73) Proprietor: Brainlab AG, 81829 München (DE)
(72) Inventor: HAIMERL, Martin, 82205 Gilching (DE); SCHUBERT, Mario, 85586 Poing (DE); GNEITING, Sabine, 82024 Taufkirchen (DE); WEGNER, Melanie, 85614 Kirchseeon (DE)
(74) Representative: SSM Sandmair
(86) International application number: PCT/EP2010/061624
(87) International publication number: WO 2011/029684

(56) References cited:
- EP-A1- 1 611 863
- EP-A1- 1 894 538
- EP-A1- 2 008 606
- EP-A2- 2 051 096
- US-A1- 2008 132 783
- DIGIOIA ET AL: "Surgical navigation for total hip replacement with the use of hipnav", OPERATIVE TECHNIQUES IN ORTHOPAEDICS, SAUNDERS, PHILADELPHIA, PA, US LNKD- DOI:10.1016/S1048-6666(00)80036-1, vol. 10, no. 1, 1 January 2000 (2000-01-01), pages 3-8, XP005183632, ISSN: 1048-6666

## Description

The present invention relates to determining the mid-sagittal plane of a pelvis which shall also be referred to in the following as a main plane. The "anatomical body part" shall also be referred simply as the "body part" for short, and the "anatomical pelvis" simply as the "pelvis". A main plane is in particular a plane of an anatomical body part which main plane is used in surgery (for example in brain or hip surgery) as a reference for defining the positions of other body parts (for example the acetabulum), in particular of other anatomical structures. A main plane is in particular a plane which can be defined by anatomical landmarks of the anatomical body part (for example the pelvis). Planes which can be defined by these landmarks and/or which are used for defining the position of the femur or acetabulum are in particular the mid-sagittal plane (MSP) or the anterior pelvic plane (APP). The main plane and the auxiliary plane are in particular not parallel and are in particular constituted to define the position of a reference system, in particular coordinate system. Generally speaking, at least two planes of the pelvis are defined in order to determine a reference system (coordinate system) with respect to which the above-mentioned position of another anatomical body part (e.g. femur or the acetabulum) can be defined. One of these planes is the aforementioned MSP the other plane can be a plane referred to here as the auxiliary plane (for example the spinae joint center plane or SJCP to be defined later) or can be a plane referred to here as the standard plane (for example the APP). Like the main plane, the auxiliary plane, and standard plane are preferably defined by landmarks of the pelvis.

As far as in the following it is referred to a main plane, this main plane is the mid-sagittal plane. As far as in the following it is referred to the auxiliary plane, it is the spinae joint center plane. As far as in the following it is referred to the standard-plane, it is the anterior pelvic plane. As far as it is referred in the following to "body part", this body part is the "pelvis".

The prior art document EP 1 611 863 A1 discloses a method for generating a three-dimensional body part model by means of a surgical navigation system. This method addresses the determination of a mid-sagittal plane of the pelvis.

EP 2 051 096 A2 discloses a method for determining a frontal plane of a pelvis. To this end, spina iliaca anterior superior points and spina iliaca posterior points are used.

US 2008/0132783 discloses a method for registering a pelvis of a subject in a lateral position. To this end, three planes are identified or calculated.

It is advantageous to determine the position of the main plane reliably and with a sufficient degree of accuracy to allow the position of the other body parts, in particular anatomical structures (for example the acetabulum) to be determined with a sufficient degree of accuracy. In accordance with a common prior-art method, the pelvis is registered in a supine position in particular for hip surgeries and then turned over to a lateral position. In the supine position, landmarks are detected by means of a pointer in order to determine the location of landmarks on the MSP and APP relative to a marker device fixed to the pelvis.

Reference is also made to US 2008/132783 A1. In accordance with this patent application, points are determined in three cardinal planes. Reference is also made to US 2003/0153829, US 2002/0077540 and WO 2005/084541.

The object of the invention is to allow a position, in particular an orientation of the mid-sagittal plane of the pelvis to be determined on the basis of the position of (main and auxiliary) points which in particular represent landmarks. Preferably, all represented landmarks can be detected (scanned) when the patient is in one position, in particular the lateral position.

The above object is solved by the subject-matter of the independent claims. The dependent claims are directed to advantageous embodiments.

As far as herein the "position" of a plane is mentioned, the term position can mean absolute location of the plane or just orientation of the plane. In particular determination of the latter is often sufficient in hip surgery since the orientation of the plane relative to the orientation of the acetabulum is of main interest for the surgeon.

One advantage of the invention is that it is not necessary to reposition the anatomical body part (for example the pelvis) and thus the patient on the table. Furthermore, the present invention relies in particular on landmarks which are easily accessible for a surgeon. In particular, points which are symmetrical to the auxiliary points with respect to the main plane and remote from the main plane (for example the ASIS point) can be detected but do not have to be detected. These symmetrical and/or remote points can be difficult to detect. Advantageously, determining the main plane does not rely on fluoroscopic images produced during surgery. Thus, the exposure of the patient and operating theatre staff to radiation is reduced.

Advantageously, the present invention can rely on position data for landmarks which can be detected to a high degree of accuracy and which are in particular not covered by soft tissue or fat. These position data are in particular detected by means of a pointer (see below).

A method in accordance with the invention is in particular a data processing method or comprises the data processing method. The data processing method is performed using technical means, i.e. a computer. The computer in particular comprises a processor and a memory in order to process the data, in particular electronically. The calculating steps described are in particular performed by a computer. Steps of determining or calculating are in particular steps of determining data within the framework of the technical data processing method, in particular within the framework of a program. A computer is in particular any kind of data processing device. A computer can be a device which is generally thought of as such, for example desktop PCs or notebooks or netbooks, etc., but a computer can be also any programmable apparatus, such as a mobile phone or an embedded processor. In particular, a computer can comprise a system (network) of "sub-computers", wherein each sub-computer represents a computer in its own right. A computer in particular comprises interfaces in order to receive data and/or to perform an analog-to-digital conversion.

The data processing method of the present invention is a method for determining the position, in particular orientation of the main plane (e.g. MSP) of the anatomical pelvis. The pelvis is in particular the pelvis of a subject (patient) who is in particular lying in a lateral position. The position, in particular orientation of the main plane is in particular determined relative to a marker device which is attached to the pelvis.

A marker device can for instance be a reference star or a pointer or one or more (individual) markers in a predetermined spatial relationship. A marker device comprises one, two, three or more markers in a predetermined spatial relationship. This predetermined spatial relationship is in particular known to a navigation system, for example stored in a computer of the navigation system.

The function of a marker is to be detected by a marker detection device (for example, a camera or an ultrasound receiver), such that its spatial position (i.e. its spatial location and/or alignment) can be ascertained. The detection device is in particular part of a navigation system. The markers can be active markers. An active marker emits for example electromagnetic radiation and/or waves, wherein said radiation can be in the infrared, visible and/or ultraviolet spectral range. The marker can also however be passive, i.e. can for example reflect electromagnetic radiation from the infrared, visible and/or ultraviolet spectral range. To this end, the marker can be provided with a surface which has corresponding reflective properties. It is also possible for a marker to reflect and/or emit electromagnetic radiation and/or waves in the radio frequency range or at ultrasound wavelengths. A marker preferably has a spherical and/or spheroid shape and may therefore be referred to as a marker sphere; markers can also, however, exhibit a cornered - for example, cubic - shape.

A "reference star" refers to a device which a number of markers, advantageously three markers, are attached to, wherein the markers are (in particular detachably) attached to the reference star such that they are stationary, thus providing a known (and advantageously fixed) position of the markers relative to each other. The position of the markers relative to each other can be individually different for each reference star used within the framework of a surgical navigation method, in order to enable the corresponding reference star to be identified by a surgical navigation system on the basis of the position of the markers relative to each other. It is thus also then possible for the objects (for example, instruments and/or parts of a body) to which the reference star is attached to be identified and/or differentiated from each other. In a surgical navigation method, the reference star serves to attach a plurality of markers to an object (for example, a bone or a medical instrument) in order to be able to detect the position of the object (i.e. its spatial location and/or alignment). Such a reference star in particular comprises a way of being attached to the object (for example, a clamp and/or a thread) and/or a holding element which ensures a distance between the markers and the object (in particular in order to assist the visibility of the markers to a marker detection device) and/or marker holders which are mechanically connected to the holding element and which the markers can be attached to.

The position of the main plane is in particular described in a reference system (coordinate system) of a navigation system, in particular a surgical navigation system (also referred to as a computer-assisted navigation system or image-guided surgery system).

A navigation system, in particular a surgical navigation system, is understood to mean a system which may comprise: at least one marker device; a transmitter which emits electromagnetic waves and/or radiation and/or ultrasound waves; a receiver which receives electromagnetic waves and/or radiation and/or ultrasound waves; and an electronic data processing device which is connected to the receiver and/or the transmitter, wherein the data processing device (for example, a computer) comprises in particular a processor (CPU), a working memory, advantageously an indicating device for issuing an indication signal (for example, a visual indicating device such as a monitor and/or an audio indicating device such as a loudspeaker and/or tactile indicating device such as a vibrator) and advantageously a permanent data memory, wherein the data processing device processes navigation data forwarded to it by the receiver and can advantageously output guidance information to a user via the indicating device. The navigation data can be stored in the permanent data memory and for example compared with data which have been stored in said memory beforehand.

According to an embodiment of the invention, absolute main point data are provided. These absolute main point data can describe the position of one (actual) main point or the position of two (actual) main points of the pelvis relative to the marker device which is in particular attached to the pelvis or of more (actual) main points. In particular, the absolute main point data describe the position of only one main point of the pelvis or the position of only two main points of the pelvis relative to the marker device. The main points are points which lie in the main plane and the position of which is defined by landmarks of the pelvis. Generally the main points can lie anywhere on the main plane. According to another embodiment, no absolute main point data are provided but instead virtual main points are calculated based on the absolute auxiliary point data and the relative point data. The virtual main points lie in the main plane and can but have not to lie in the body part. These calculated virtual main points can be used for the further determination, in particular calculation in accordance with the described inventive method in the same way as the actual main points described by the absolute main pint data. According to a further embodiment both the provided actual main points and the calculated virtual main points are used for the further determination in accordance with the invention.

A landmark is a defined position of an anatomical characteristic of an anatomical body part which is always identical or recurs with a high degree of similarity in the same anatomical body part of multiple patients. Typical landmarks are for example the anterior superior iliac spine (ASIS) points or the tips of the dorsal process of a vertebra. The points (main points or auxiliary points) can represent such landmarks. A landmark which lies on (in particular on the surface of) a characteristic anatomical structure of the body part can also represent said structure. The landmark can represent the anatomical structure or only a point or part of it. For instance, a landmark can also lie on the anatomic structure which is in particular a prominent structure. An example of such an anatomic structure is the posterior aspect of the iliac crest. Other landmarks include a landmark defined by the rim of the acetabulum, for instance by the center of the rim. Another example is where a landmark represents the bottom or deepest point of an acetabulum, which is derived from a multitude of detection points. Thus, one landmark can in particular represent a multitude of detection points. As mentioned above, a landmark can represent an anatomical characteristic which is defined based on a characteristic structure of the body part. Additionally, a landmark can also represent an anatomical characteristic defined by a relative movement of two body parts, such as the rotational center of the femur when moved relative to the acetabulum.

A detection point is in particular a point on the surface of the anatomical structure which is detected, for example by a pointer.

Absolute auxiliary point data are also provided in accordance with the invention. These absolute auxiliary point data describe the position of at least one auxiliary point of the pelvis relative to the marker device. Unlike the main points, an auxiliary point lies outside the main plane. If there is more than one auxiliary point, the auxiliary points lie in particular in the so-called auxiliary plane (already mentioned above), as will be explained in more detail below. The auxiliary points represent the position of the anatomical characteristics and are in particular (directly or indirectly) defined by landmarks. Both the main points and the auxiliary points are in particular, but not necessarily, points lying on the surface of the pelvis. As mentioned above, the main points and auxiliary points can also be defined indirectly, as will be explained in more detail below, for instance by the rim of the acetabulum or by a rotation center or by landmarks which are symmetrical relative to a plane (for example the main plane or auxiliary plane), again to name but a few examples of indirect definitions of auxiliary points or main points.

According to one advantageous embodiment of a determination method in accordance with the invention, the position of the main points and/or auxiliary points (which represent landmarks) can be detected by a step of contacting the pelvis with the above-mentioned pointer which is handled by a medical assistant and brought into contact with the pelvis. This optional detection step allows the absolute main point data and absolute auxiliary point data to be provided and in particular does not form a part of the claimed data processing method but can form a part of a general determination method for determining the position of the main plane. The step of detecting in particular does not encompass or comprise an invasive step representing a substantive physical intervention on the body which requires professional medical expertise to be carried out and/or which entails substantial health risk even when carried out with required professional care and expertise. The step of detecting does in particular not comprise or encompass a surgical step. The step of detecting does in particular not comprise or encompass a step for treatment of a human or animal body by surgery or therapy. The step of providing the absolute main point data and absolute auxiliary point data includes in particular a step of receiving the absolute main point data and absolute auxiliary point data by the data processing method. The received data have been in particular generated by the detection step. The detection step is in particular not part of the data processing method but is optionally part of the determination method in accordance with the invention. As mentioned above, a multitude of detected points (detection points) on the surface of the pelvis can result in the position of just one landmark and therefore just one main point and/or auxiliary point being detected. A multitude of detection points are for example necessary in order to determine the deepest point (fossa) in the acetabulum which is a landmark. Another example is when a multitude of detection points are necessary in order to define several positions of the femur relative to the acetabulum. This allows the center of rotation of the relative movement of the femur to be determined. This center of rotation is then a landmark.

The data processing method of the present invention is directed to data processing and in particular does not include steps relating to contacting an anatomical structure.

Relative point data are also provided in accordance with the invention. These relative point data constrain the possible position of the main plane relative to the at least one auxiliary point, in particular relative to only one of the one or more auxiliary points and/or relative to only two of the auxiliary points. The relative point data can in particular constrain the possible positions between a particular auxiliary point of the one or more auxiliary points and another point referred to as the virtual auxiliary point, which is not included in the absolute auxiliary point data. This virtual auxiliary point is in particular a point which is symmetrical to the particular auxiliary point of the one or more auxiliary points with regard to the main plane. Thus, the possible positions of the main plane relative to the at least one auxiliary point are in particular constrained by constraining the possible positions between the at least one auxiliary point and the virtual auxiliary point and/or between the at least one auxiliary point and a virtual main point (see below). An example of such a constraint is that the auxiliary point and its corresponding (symmetrical) virtual point must have a certain distance. A further example of the relative point data is that these data describe an angle between two lines defined by at least three points, at least one of these three points can be a main point (virtual or actual main point). Thus also positional relationships, in which (virtual or actual) main points are involved, can represent constraints for the possible positions of the main plane relative to the at least one auxiliary point. The absolute main point data describe in particular the position of one or two actual main points while they do not describe the position of the virtual main points. The virtual main points are in particular determined, in particular calculated by means of the data processing method. The actual main points are in particular received by the data processing method as mentioned above. Correspondingly, the absolute auxiliary point data describe the position of at least one actual auxiliary point while they do not in particular describe the position of virtual auxiliary points. The virtual auxiliary points are in particular determined, in particular calculated by means of the data processing method.

The constraints described by the relative point data are in particular represented by one or two or more scalar values. These scalar values are in particular used for describing a positional relationship between the main plane and a zero-dimensional or one-dimensional geometrical object, such as a point or line, or between two such geometrical objects. The constraints given by the relative point data are in particular incomplete, i.e. do not allow the exact position of the main plane relative to the at least one auxiliary point to be calculated in a reference system (coordinate system) based only on the position of the at least one auxiliary point and the relative point data. Additional information is necessary. This additional information is for instance the absolute main point data and/or absolute auxiliary point data and/or the pelvis data. The relative point data are in particular incomplete in that at least the position of one main point and at least the position of one auxiliary point are necessary in addition to the relative point data, in order to calculate a position of the main plane. Thus, the relative point data can directly constrain the possible positions of the main plane relative to the at least one auxiliary point by describing a positional relationship (for example a distance or angle) between the at least one auxiliary point and the main plane. The relative point data can also indirectly constrain the positions of the main plane relative to the at least one auxiliary point by describing a positional relationship (for example a distance or angle) between at least one auxiliary point and at least one virtual auxiliary point.

In particular, the relative point data can include but do not need to include geometrical constraints which describe a predetermined positional relationship between planes of the anatomical pelvis, in particular between the auxiliary plane and the main plane and/or between the main plane and the standard plane and/or between the standard plane and the auxiliary plane. The relative point data in particular do not describe that this predetermined positional relationship is a perpendicular relationship. Nevertheless, the constraints allow the number of possible positions to be restricted. The relative point data comprise in particular one scalar value, in particular only one scalar value, if the absolute point data comprise two main points. The relative point data also in particular comprise two scalar values, in particular only two scalar values, if only one main point is provided by the absolute point data. Examples of scalar values are distances and angles.

The relative point data are in particular stored in a data storage (e.g. RAM, ROM, or any database). The relative point data can be generated, preferably before the surgery starts, in particular outside the operating theatre. The relative point data are in particular based on at least one x-ray image of the pelvis. The relative point data are in particular only based on two-dimensional x-ray images. In particular, the relative point data describe constraints which can be derived from (for example based on) a two-dimensional image, in particular only one two-dimensional image (but also possibly two or three or more), for example only one x-ray image. Thus, the relative point data in particular describe positional relationships between geometrical objects, wherein said relationships are present in two dimensions (for example in an image plane). In this way, the data processing method of the present invention can use data which are easily obtained and in particular available before surgery. Thus, it is not necessary to take additional x-ray images, in particular during surgery. It is a particular aspect of the invention that data available before surgery are used to reduce the workload and so aid the surgeon when gathering data from the pelvis by means of a pointer. Furthermore, uncertainties in the position of the determined main plane due to a change in the location of the patient (due to being turned over from the supine position to the lateral position) can be avoided. In the inventive method, relative point data is the (shortest) distance from an auxiliary point to the main plane. Another example is where there are two auxiliary points which define a line. The relative point data can then describe the distance from one of the auxiliary points to the main plane when following the line and/or can define the angle of the line relative to the main plane. The relative point data may also be based on anatomical knowledge, for instance based on generic or statistical models of the pelvis. Based on these models, for instance distances or angels are derived which are used as relative point data. Furthermore, it is also possible to use, in particular before surgery, a mechanical tool in order to measure the relative point data. For instance, the distance between the two ASIS points may be determined by using a mechanical tool and then the resulting distance may be entered into the system as relative point data. The relative point data can not just describe constraints for the possible positions of the main plane relative to the at least one actual auxiliary point but can in addition describe constraints for the possible positions of the main plane relative to at least one virtual auxiliary point.

As mentioned above, only a minimum number of auxiliary points, preferably but not obligatory in combination with an actual main point can be used as basis for the calculation of the position of the main plane. Thus, there is a minimum of information needed for calculating the position of the main plane. However, of course, more than this minimum information can be used as a basis for the calculation. In particular several actual main points or several actual auxiliary points can represent the basis for the calculation. In that case, a possible error in the calculation of the position of the main plane may be reduced by using this additional information in accordance with general known error reduction methods in case of more information as necessary is available.

The step of calculating the position of the main plane relative to the marker device preferably includes a step of determining a virtual auxiliary point and/or a virtual main point as described above. The position of the main plane is then determined on the basis of the determined virtual main point and/or virtual auxiliary point. There is thus preferably an intermediate step of determining at least one virtual auxiliary point and/or at least one virtual main point (see below).

The majority and in particular preferably all of the actual auxiliary points used for calculating the position of the main plane are preferably outside the main plane but on the same side of the main plane. This is particularly advantageous if the main plane divides the pelvis into parts of at least approximately the same size. This is for instance the case with the mid-sagittal plane (MSP). In this way, it is possible to ensure that the auxiliary points are easily accessible for the surgeon and that the data provided for the data processing method are reliable data, since they were easily and clearly accessible for the surgeon. Another embodiment will be described below, in which the auxiliary points are provided on both sides of the main plane but preferably close to the main plane.

Preferably, the absolute auxiliary point data are used to determine (calculate) at least one additional main point which lies in the main plane and which is the above-mentioned virtual main point. A virtual main point is not included in the absolute main point data, but does lie in the main plane. A virtual main point is in particular not based on the detection of a landmark by means of a pointer. If there are in particular no actual main point or only one or only two actual main points, then there is not enough information available to determine the position of the main plane on the basis of the absolute main point data. Therefore, in accordance with one embodiment of the invention, a particular auxiliary point is used to determine at least one virtual main point. One or more or all of the one or more auxiliary points can be a particular auxiliary point. For the calculation of the virtual main points also already calculated virtual main points and/or actual main points may be used.

The particular auxiliary point can also be used to determine (calculate) at least one additional auxiliary point which lies outside the main plane and which is the above-mentioned virtual auxiliary point. A virtual auxiliary point is not included in the absolute auxiliary point data. A virtual auxiliary point is in particular not based on the detection of a landmark by means of a pointer. The virtual auxiliary point can have a defined positional relationship, in particular a symmetrical relationship with respect to the main plane and preferably also with respect to the particular auxiliary point. The virtual auxiliary point is for example a point which is symmetrical to the particular auxiliary point with respect to the main plane. As mentioned above, the position of the main plane can then be determined on the basis of the particular auxiliary point and the corresponding (symmetrical) virtual auxiliary point. Thus, there is then enough information available to determine the position of the main plane on the basis of the virtual auxiliary point.

As mentioned above, the relative point data include at least one scalar value. This at least one scalar value describes a positional relationship between an auxiliary point and the main plane (for instance, the (shortest) distance from the auxiliary point to the main plane). The scalar value can also describe a positional relationship between two auxiliary points, one of which is a virtual auxiliary point. This virtual auxiliary point is in particular a point at a position which is symmetrical to the other (actual) auxiliary point with respect to the main plane. An example of this is the sinistral and dextral anterior superior iliac spine (ASIS) point. For instance, the dextral ASIS point is the actual auxiliary point and the sinistral ASIS point is the virtual auxiliary point. This situation applies in particular if the patient is lying on their sinistral side. The scalar value can in particular describe the distance between two fossa points of the acetabulum (i.e. the deepest point of the acetabulum). The relative point data describe this distance according to an embodiment of the present invention. One of the two fossa points is an actual auxiliary point and the other one is a virtual auxiliary point. Thus, the determination method can generate the absolute auxiliary point data e.g. by detecting the position of one of the fossa points by means of a pointer. The generated absolute auxiliary point data can then be received by the data processing method. The distance between the two fossa points which is called inter-fossa distance can be deduced from an x-ray image. The inventors of the present invention have found that deducing the distance from an x-ray image is not obligatory. The inter-fossa distance within males and within females are rather constant. Therefore, a particular distance for a male or for a female can be assumed. This distance represents an example for relative point data described by a scalar value. Typical values for inter-fossa distance for males is 116 mm. Typical values for inter-fossa distance for females is 125 mm. For Asian people the values are a bit lower (114 mm inter-fossa for males and 122 mm for females). In particular an inter-fossa distance of 116 mm +/- 7 mm for males and 125 mm +/-8 mm has been determined by the inventors. In particular, an inter-teardrop-distance of 112 mm +/- 6 mm for males and an inter-teardrop-distance of 121 mm +/- 8 mm for females has been determined by the inventors. Thus, preferably, the predetermined value for inter-fossa distance for males is set to be higher than 109 mm and/or smaller 123 mm for males and/or set to be higher than 118 mm and/or lower than 133 mm for females. In particular, the predetermined value for an inter-teardrop-distance is set to be higher than 106 mm and/or lower than 118 mm for males and/or higher than 113 mm and/or lower than 129 mm for females.

Thus, according to an advantageous embodiment of the present invention, the inter-fossa distance is a predetermined value which is provided according to a step of the data processing method, in particular received by the data processing method as relative point data (e.g. from a database). In particular, this distance can be stored in a database and can be received from the data processing method by accessing the database. Besides the inter-fossa distance, the inventors of the present invention have also found that the inter-teardrop distance is rather constant. Therefore according to another advantageous embodiment, the inter-teardrop distance is used as relative point data. In particular, there is a fixed relationship between inter-teardrop distance and inter-fossa distance which can be used if the position of one of the fossa points is described by the absolute auxiliary point data. In particular, the teardrop distance is smaller than the inter-fossa distance by a predefined difference which difference is in particular larger than 1 or 2 mm and smaller than 3 or 4 mm. In particular, the difference is about 2 to 3 mm. The inter-teardrop distance is generally used by a physician. The physician determines the inter-teardrop distance by measuring a distance in an x-ray image of the patient. Preferably, the data processing method includes a step of providing the difference between the inter-teardrop distance and the inter-fossa distance so that one of the two distances can be calculated if the other one is provided (in particular received by the data processing method). For instance the physician measures the inter-teardrop distance from a x-ray image of the patient and inputs the distance into the data processing method. Thus the inter-teardrop distance is received by the data processing method and the inter-fossa distance can be calculated from the inter-teardrop distance based on the aforementioned known difference. If no measurement of the distance is performed, according to an aspect of the invention, a predetermined value for the inter-teardrop distance and/or inter-fossa distance is used by the data processing method, in particular typical value is used by the data processing method. In other words, the data processing method includes the step of providing a predetermined value for the inter-teardrop distance and/or inter-fossa distance according to an embodiment.

Generally, according to an advantageous aspect of the invention, properties, in particular distances and angles of the anatomical body part (in particular the pelvis) are used for defining the relative point data which properties are at least approximately constant for particular types of human beings (in particular for particular types of racial populations (e.g. Asian, Caucasian or African) and/or for a particular type of gender (male or female). Preferably those properties are used which are describable by a scalar value (e.g. distance or angle) and which are at least approximately constant, i.e. the variation of the scalar value is low or zero. Low variation in the meaning of the present invention is in particular if the standard deviation from a mean scalar value is lower than 5%, 2% or 1% of the scalar value which describes the property. More preferably, low variation means that the variation of the scalar value results in a variation of less than 2 degree for the position (orientation) of the acetabulum and/or femural shaft. The position (in particular orientation) is preferably described by an angle with respect to a plane, in particular standard plane (see below). Preferably, a variation of the scalar value is considered to be a low variation, if the variation of the scalar value results in a variation of the angle of less than 5 degrees, in particular less than 2 degrees, in particular with respect to a target position (in particular target orientation) which is in particular also described by an angle with respect to a plane (in particular the standard plane). By referring to general properties which are approximately constant at least for different types of human beings, further analysis of the anatomical body parts by means of analytical devices (like x-ray or CT or MRT) can be avoided and costs and time can be spared. Furthermore, radiation to the patient and to the medical team can be reduced.

According to a further advantageous embodiment, the aforementioned properties of the anatomical body part which are at least approximately constant for particular types of human beings are described to be a function of other properties of the human being. For instance, the inter-fossa distance or the inter-teardrop distance can be described to be a function of the length of the femur or the body height. In this way, it can be possible to determine the data used as relative point data more exactly and more individually by varying an approximately constant anatomical property (in particular described by a scalar value) in accordance with the function.

Where the provision of an "auxiliary point" is mentioned here, it is meant that an actual auxiliary point is provided unless otherwise specified, i.e. an auxiliary point included in the absolute auxiliary point data. In all other cases it may be both (virtual and actual auxiliary point). Where the provision of a "main point" is mentioned here, it is meant that an actual main point is provided unless otherwise specified, i.e. a main point included in the absolute main point data. In all other cases, it may be both (virtual and actual main point). In particular, the absolute main point data describe only those positions of main points which are used to calculate the position of the main plane. In particular, the absolute auxiliary point data describe only those positions of auxiliary points which are used to calculate the position of the main plane. As the actual main and/or auxiliary points preferably do, the virtual main and/or auxiliary points in particular (but not necessarily) represent landmarks. In particular, as far as herein the determination (calculation) of a position of a plane is concerned and a reference is made to main points, if not otherwise specified, this means preferably but not obligatory that not only actual main points can be used for the determination (calculation) but also virtual main points can be used (in addition or exclusively). The same applies if a positional relationship between a main point and any other geometric object (e.g. point or plane) is concerned.

In particular, as far as herein the determination (calculation) of a plane is concerned and a reference is made to auxiliary points, if not otherwise specified or clear from the description, this means preferably but not obligatory that not only actual auxiliary points can be used for the determination (calculation) but also virtual auxiliary points. The same applies if a positional relationship between an auxiliary point and another geometric object (e.g. point or plane) is concerned.

Preferably, the position of the actual auxiliary point in combination with the relative point data allows the position of the virtual auxiliary point to be determined. If the virtual auxiliary point is symmetrical to the main plane with respect to the actual auxiliary point, determining the position of the virtual auxiliary point allows the position of the main plane to be determined.

Also the virtual auxiliary points may be determined indirectly based on the detection of points outside the body part. For instance, the plane on which the patient is lying is determined by using a pointer. Assuming further, the patient is lying in lateral position, e.g. its sinistral ASIS point is in contact with the plane. Furthermore, assuming, the relative point data describe the distance between the sinistral and the dextral ASIS point, then the determined location of the plane on which the patient is lying, allows to calculate, based on the relative point data and the position of the dextral ASIS point (which is provided by the absolute auxiliary point data), the position of the virtual sinistral ASIS point. This virtual auxiliary point may be used for the calculation of the planes in the same manner as the aforementioned actual auxiliary points.

The present invention also preferably uses anatomical knowledge concerning the pelvis. For example, as mentioned above, the relative point data can use this knowledge when referring to statistical models of the pelvis in order to determine distances or angles. For example, the above-mentioned inter-fossa or inter-teardrop distance represent anatomical knowledge (found by the inventors) based on which the relative point data are provided.

Furthermore, the anatomical knowledge is preferably used to provide body part data (also referred to as pelvis data). These body part data (pelvis data) constrain the possible relative (anatomical) position between landmarks of the pelvis and/or between the landmarks and the main plane. For instance, body part data (pelvis data) describe that one of the landmarks is more anterior or more posterior or more distal or more proximal or more cranial or more caudal than another landmark. The body part data (pelvis data) can also constrain the possible relative (anatomical) positions between (prominent) anatomical positions of (prominent) anatomical structures such as a crest or an anatomical plane (for example the mid-sagittal plane) or between such structures and landmarks. Thus, the body part data can also be defined in the form of inequality constraints.

In order to use the pelvis data provided, landmark data are preferably provided which link at least some of the main points and/or auxiliary points to the landmarks of the pelvis. The term "at least some" here means in particular at least two or at least three of the points, which can be main points and/or auxiliary points. In other words, the landmark data inform the navigation system as to which (actual or virtual) (main or auxiliary) point represents which landmark. This information is given for at least some of the points.

It is possible, when attempting to determine the position of the main plane on the basis of the provided absolute main point data, the provided auxiliary point data and the provided relative point data, for this attempt to result in more than one possible solution. If more than one solution does result, the provided pelvis data and the provided landmark data are in particular used to discount one or more of the solutions which are not in line with the pelvis data, i.e. which do not correspond to the anatomy of the pelvis and can therefore be discounted. This allows one of the possible solutions for the position of the main plane to be selected on the basis of the provided pelvis data and the provided landmark data. In other words, the solution which is in line with the constraints given by the pelvis data is selected.

As mentioned above, it is not obligatory to provide absolute main point data. However, in that case, preferably at least one virtual main point is calculated based on the absolute auxiliary point data. This calculated at least one virtual main point may be used in the further calculation in the same way as described below for the one or two actual main points. In other words, the provision of actual main points can be replaced by the calculation of virtual main points.

According to a particular embodiment of the present invention, the absolute main point data describe a position of only one main point of the pelvis relative to the marker device or only one virtual main point has been calculated or can be calculated, then two (actual or virtual) main points are in particular missing in order to determine the main plane since only one (actual or virtual) main point is available for the calculation. In this case, the absolute auxiliary point data in particular describe the position of at least two auxiliary points of the pelvis relative to the marker device. As mentioned above, the auxiliary points are outside the main plane. In particular, the actual auxiliary points are on the same side of the main plane. In this way, the data processing method can be based on data which can be easily obtained by a surgeon. In particular, the absolute auxiliary point data describe the position of only two auxiliary points.

In accordance with another embodiment, the absolute main point data describe the position of only two main points of the pelvis relative to the marker device or two virtual main points have been calculated (or can be calculated) or there is one actual main point and one virtual main point, then one additional (virtual or actual) main point is in particular missing in order to determine the main plane since only two (actual or virtual) main points are available for the calculation. In this case, the absolute auxiliary point data preferably describe the position of at least one auxiliary point of the pelvis relative to the marker device, in particular the position of only one auxiliary point of the pelvis relative to the marker device. The auxiliary points are in particular outside the main plane and in particular on the same side of the main plane. Thus, the data processing method can in this case again be performed on the basis of data which are easily generated.

In accordance with another embodiment, the relative position data describe at least one constraint, in particular only one constraint for the possible positions of the main plane relative to the at least one auxiliary point, if the absolute main point data describe the position of two main points, in particular of only two main points. In this case, there is in particular only one auxiliary point.

In accordance with another embodiment, the relative position data describe at least two constraints for the possible positions of the main plane relative to the at least one auxiliary point, if the absolute main point data describe the position of only one main point. In this case, the absolute auxiliary point data describe the position of at least two auxiliary points, in particular of only two auxiliary points.

As mentioned above, the main plane is the mid-sagittal plane in accordance with the invention.

In the field of navigated surgery, in particular computer-assisted surgery or image-guided surgery, a reference system (for example a coordinate system) in which the pelvis is located is preferably determined. In order to determine such a coordinate system, two planes defined by the shape of the pelvis are preferably determined. One of these planes is in particular the mid-sagittal plane; the other plane can be the anterior pelvic plane or - as will be explained in more detail below - a spinae joint center plane as a new reference plane referred to as "SJCP". The spinae joint center plane is defined by auxiliary points. In summary, there are preferably at least two planes which have to be determined in order to have an adequate basis for providing navigation information to the surgeon. These two planes and the resulting reference system are in particular used to define the position and in particular the orientation of the acetabulum, in particular for hip surgery.

The present invention also relates to a data processing method which includes the above-mentioned data processing method and not only allows the position of the main plane of the pelvis but also an auxiliary plane of the pelvis to be determined.

The data processing method for determining the main plane and the auxiliary plane preferably uses the above-mentioned data processing method in order to determine the main plane. The above-mentioned data processing method will therefore be referred to as the first data processing method. The data processing method for determining both the main plane and the auxiliary plane will be referred to as the second data processing method. The second data processing method uses the following approach for determining the auxiliary plane. Absolute auxiliary point data are provided which describe the position of at least two of the auxiliary points of the pelvis relative to the marker device. Thus, contrary to the first data processing method, there is preferably a minimum of two auxiliary points, the position of which is known from the absolute auxiliary point data. The auxiliary points are preferably outside the main plane. The auxiliary points are in particular on the same side of the main plane. Preferably, at least one of the at least two auxiliary points is used for calculating the position of the main plane. In accordance with another embodiment, the at least two auxiliary points are used for calculating the position of the main plane. Data are also provided which are referred to as relative auxiliary plane data. These auxiliary plane data describe the positional relationship between the auxiliary plane and the main plane. Furthermore, it is assumed that the auxiliary points lie within the auxiliary plane.

In accordance with the second data processing method, the provided relative auxiliary plane data are preferably used to calculate the position of the auxiliary plane on the basis of the assumption that the at least two auxiliary points lie within the auxiliary plane. In other words, the auxiliary plane is determined in such a way that it includes the at least two auxiliary points and fulfils the predetermined positional relationship with respect to the main plane, said relationship being known from the relative auxiliary plane data.

One example of the predetermined positional relationship is a particular angle between the main plane and the auxiliary plane. The angle can in particular be within the range of 30° to 150°. The predetermined positional relationship can in particular be such that the auxiliary plane is perpendicular to the main plane.

The invention is also directed to a third data processing method which comprises the second data processing method explained above. The third data processing method is a method for determining the position of the main plane and of a standard plane, wherein the position of the auxiliary plane has been determined by the second data processing method. The auxiliary points lying in the auxiliary plane are landmarks of the pelvis. Examples of auxiliary points will be given below.

In accordance with the third data processing method, relative standard plane data are also provided. The relative standard plane data describe the expected relative positional relationship between the auxiliary plane and the standard plane. The expected relative positional relationship is a positional relationship which can correspond to an average positional relationship derived from a statistical analysis of the positional relationship between the auxiliary plane and the standard plane for a plurality of different pelvises. In particular, the average positional relationship which results from this statistical analysis represents the expected positional relationship. Any kind of (statistical) method for determining an average can be applied, for instance the arithmetic mean or the median or the mode. A generic model of the pelvis can also be used to determine the relative positional relationship between the auxiliary plane of the generic model and the standard plane of the generic model. This positional relationship also represents an example of an expected positional relationship.

In accordance with the third data processing method, the position of the standard plane is determined on the basis of the position of the auxiliary plane (determined using the second data processing method) and on the basis of the relative standard plane data. For instance, the relative standard plane data describe an angle between the position of the auxiliary plane and the position of the standard plane. Thus, the determined position of the auxiliary plane allows the position of the standard plane to be calculated on the basis of the standard plane data. A standard plane is in particular a plane with respect to which the position of the femoral shaft and/or the position (in particular orientation) of the acetabulum is (usually) described.

As mentioned above, the auxiliary points can represent landmarks in any of the data processing methods described above (i.e. the first, second and/or third data processing method). Examples of landmarks represented by the auxiliary points include the sinistral ASIS point or dextral ASIS point. The auxiliary points can alternatively or additionally represent a landmark which is defined by the acetabulum and/or by a partial (surface) point of the acetabulum. The auxiliary point can then in particular be the rotational center or the deepest part of the acetabulum or the most proximal part of the acetabulum. The main plane in any of the data processing methods described above (i.e. the first, second or third data processing methods) can for example be the mid-sagittal plane. The standard plane as described with respect to the third data processing method can for example be the anterior pelvic plane. The relative standard plane data described in connection with the third data processing method describe in particular the angle between the anterior pelvic plane and the auxiliary plane. The auxiliary plane (for example the SJCP) is in particular a plane described by the sinistral and dextral ASIS point and an auxiliary point defined by the acetabulum or a point or part of the acetabulum. The auxiliary plane is in particular such that the auxiliary plane and the anterior pelvic plane intersect each other along a line connecting the sinistral and the dextral anterior superior iliac spine landmarks of the pelvis. The present application is also directed to an independent invention which is directed to a data processing method which relies on the relative standard plane data which describe the relative position between the anterior pelvic plane (APP) and the spinae joint center plane (SJCP). Thus, preferably any data processing method or system or computer etc. is a subject-matter of the present invention which relies on this kind of standard plane data. The inventors of the present invention have first found that the APP and SJCP have a statistically stable relative positional relationship which may be used in any kind of data processing methods which processes data related to the pelvis. Therefore, the present application is also directed to such an independent invention which may be a subject-matter of a later divisional application. These data processing methods are in particular directed to a calculation of positions, in particular orientations of planes or parts or points of the pelvis for computer aided navigation in surgery (image guided surgery). Such a data processing method is in particular as follows: A data processing method for determining the position of a plane or a part or a point of pelvis which comprise the steps of: providing relative standard plane data which describe the expected positional relationship between the SJCP and the APP; and determining the position of the APP on the basis of the position of the SJCP and the relative standard plane data.

The following embodiment can be combined with the aforementioned methods or can also represent an independent embodiment of the invention. In accordance with this embodiment, additional auxiliary points are determined which are in particular symmetrical to another auxiliary point if the main plane is taken as the plane of symmetry. Said other auxiliary point (which corresponds to the additional auxiliary point) is referred to as the symmetrical auxiliary point. The (at least one) symmetrical auxiliary point is included in the absolute auxiliary point data, whereas the (at least one) additional auxiliary point is not included. As an independent embodiment of the invention, the (at least one) symmetrical auxiliary point is provided in a step of the independent data processing method. In accordance with this embodiment, relative auxiliary point data are provided. These relative auxiliary point data describe at least one constraint for possible positions of the additional auxiliary point relative to its corresponding symmetrical auxiliary point. When the symmetrical auxiliary point is on one side of the main plane, the corresponding additional auxiliary point is in particular on the other side. In accordance with this embodiment, candidate point data are provided. The candidate point data describe the position of a candidate point with respect to the marker device. These candidate points are candidates for the additional auxiliary point. New candidate point data which describe the position of new candidate points relative to the marker device are preferably provided in steps. All the candidate points provided are preferably on the same side of the main plane. The new candidate point data are preferably received in steps, such that candidate point data are provided in steps. A candidate point received in the current step is referred to as the current candidate point. In accordance with this embodiment, the next step consists of checking whether the position of the current candidate point (i.e. the new candidate point) complies with the at least one constraint. The at least one constraint is described by the relative auxiliary point data as mentioned above. An example of the constraint is in particular a distance between the symmetrical auxiliary point and its corresponding additional auxiliary point.

After the checking step, the current candidate point (new candidate point) is preferably accepted as an additional auxiliary point if the position of the current candidate point complies with the at least one constraint defined by the relative auxiliary point data, i.e. if the position of the current candidate point is a possible position. If the position of the current candidate point does not comply with the at least one constraint, then the aforementioned steps (of providing and checking) are repeated. Since the additional auxiliary point is symmetrical to the symmetrical auxiliary point with respect to the main plane, the position of the main plane can be determined on the basis of the additional auxiliary point and the symmetrical auxiliary point. The new candidate point data are preferably generated by detecting a point which contacts an additional anatomical structure which is symmetrical to another anatomical structure (referred to as the symmetrical structure). This structure is in particular a prominent structure (for example a rim or crest). A point detected on the symmetrical structure corresponds to the symmetrical auxiliary point. A plurality of points detected on the additional anatomical structure are candidate points for the additional auxiliary point. During the movement of the pointer along the additional anatomical structure, new candidate points are generated in steps. In order to guide the user during the movement of the pointer, indication data are preferably provided. Both the additional auxiliary point and the symmetrical auxiliary point are preferably determined on the basis of the above-mentioned detection points (representing detected landmarks) and in particular not on virtual auxiliary points.

In accordance with another embodiment, the aforementioned method comprises additional steps which relate to generating indication data. These indication data are in particular based on the result of the checking step and indicate whether the position of the candidate point complies with the at least one constraint. These indication data are preferably used by an indication method which comprises the aforementioned data processing method and which generates indication signals on the basis of the indication data in order to inform a user (surgeon) as to whether the current candidate point complies with the constraint. Preferably, a deviation between a scalar value describing the constraint (for instance a distance) and a scalar value describing the position of the current candidate point relative to a main point or the particular auxiliary point is calculated. The result of the calculation is then preferably part of the indication data and is thus also indicated to the user as part of the indication signal. A surgeon can use the above-mentioned data processing method as an assistance in finding an auxiliary point when scanning the pelvis with a pointer.

The present invention is also directed to a program which, when it is running on a computer or is loaded onto a computer, causes the computer to perform the data processing method as described in any one of the preceding embodiments. The present invention is also directed to a program storage medium (for instance a CD, ROM, RAM, harddrive, etc.) on which the program is stored. The present invention also relates to a computer on which the program is running, by which the program is executed or on which the program is loaded. This computer in particular comprises a memory which stores the program. As a further example, a signal wave may also carry the program for example during a download process when downloading the program via the internet.

The present invention is also directed to a navigation system for computer-assisted surgery. This navigation system preferably comprises the aforementioned computer for processing the data provided in accordance with the data processing method as described in any one of the preceding embodiments. Preferably, the navigation system comprises a detection device for detecting the position of the detection points which represent the main points and auxiliary points, in order to generate detection signals and to supply the detection signals generated to the computer such that the computer can determine the absolute main point data and absolute auxiliary point data on the basis of the detection signals received. In this way, the absolute point data can be provided to the computer. The navigation system also preferably comprises a user interface for receiving the calculation results from the computer (for example the position of the main plane, the position of the auxiliary plane and/or the position of the standard plane). The user interface provides the received data to the user as information. Examples of a user interface are a monitor or a loudspeaker. The user interface can use any kind of indication signal (for example a visual signal, an audio signal and/or a vibration signal).

Where data are described here as being "provided", this means that they are ready for use by the method in accordance with the invention. The data can achieve this state of being "provided" by for example being detected or captured (for example by a detection device) or by being inputted (for example via interfaces) or by being determined on the basis of input signals or detection signals or input data. The data can also have this state by being stored in a memory (for example a ROM, CD and/or hard drive) and thus ready for use within the framework of the method in accordance with the invention.

The expression "providing data" encompasses (within the framework of a data processing method) in particular that the data are determined by the data processing method or program. The meaning of "providing data" in particular encompasses also that the data are received by the data processing method or program (e.g. from another program or a data storage), in particular to further process the data by the data processing method or program. Thus "providing data" can mean for instance to wait for a reception of data and to receive the data. The received data can be for instance inputted by the interface. "Providing data" can also mean that the data processing method or program performs steps to (actively) acquire the data from a data source, for instance a data storage (for instance ROM, RAM, data base, hard disk etc.) or via the interface (for instance from another computer or a network). The data can achieve the state of being "ready for use" by performing a further step before the providing step. According to the further step, the data are generated for providing the data. In particular the data are detected or captured (for example by an analytical device). Alternatively or additionally, the data are input according to the further step, for instance via interfaces. In particular, the generated data can be input (for instance in the computer). According to the further step (before the providing step), the data can also be provided by performing the further step of storing the data in a data storage (for example a ROM, RAM, CD and/or hard drive) and thus ready for use within the framework of the method or program in accordance with the invention. The step of providing in particular does not encompass or comprise an invasive step representing a substantial physical intervention on the body which requires professional medical expertise to be carried out and/or which entails substantial health risk even when carried out with the required professional care and expertise. The step of providing does in particular not comprise or encompass a surgical step. The step of providing does in particular not comprise or encompass a step for treatment of a human or animal body by surgery or therapy. The same applies for any steps directed to the determination of data.

In order to detect points on a plane, for instance actual main points and/or actual auxiliary points, a tool called plane point detection tool can be used. The present invention is also directed to the use of such plane point detection tools in combination with the above described method and navigation system.

In particular, an aspect of the present invention is directed to such a tool called plane point detection tool. The plane point detection tool (also just called "tool" in the following) is for detecting the position of at least one actual main point and/or at least one actual auxiliary point. The plane point detection tool is preferably constituted to be arranged in a plane of the anatomical body part (e.g. main plane or auxiliary plane).

It may comprise:
a proximal side constituted to be fitted to a patient by contacting the patient along a first direction of length-wise extension of the tool, the proximal side is constituted to be able to be aligned with a plane of an anatomical body part along the first direction of length-wise extension, the direction in which the proximal side faces and along which the tool is flexibly bendable for bringing the tool into contact with the patient being a third direction of extension of the tool;
a distal side which comprises at least one pointer insertion member comprising a tapered recess for inserting the tip of a pointer, the at least one pointer insertion member being prominently positioned in a first section of the distal side which is, in the direction of length-wise extension, closer to a first end of the plane point detection tool than to a second end; wherein the distal side further comprises a second section having a flat surface and being closer to the second end than to the first end;
both the proximal side and distal side extending in the first direction and in a second direction of extension, the second direction being traverse to the first direction,
the tool being flexibly bendable in the third direction while being rigid in the second direction, the second direction being a direction of width-wise extension of the tool, the width-wise extension of the tool being smaller than the length-wise extension.

The aforementioned tool can in particular be part of the navigation system of the present invention and can in particular be used in combination with the method, in particular the data processing method of the present invention. In particular, the tool is used for generating detection signals by means of a pointer which contacts the bottom of the tapered recess of the plane point detection tool. The tapered recess is in particular constituted such that the pointer can be pivoted during that contact.

The plane point detection tool preferably comprises a front side and a back side. The front side is close to the user (e.g. member of the medical staff, in particular surgeon) who inserts the pointer into the at least one tapered recess of the tool. The backside of the tool is to be attached to the patient and is therefore called proximal side. Correspondingly, the front side is called distal side. According to a preferred embodiment for using the tool, the tool is attached to the backside of the pelvis in the region of the sacrum along the midsagittal plane. That is, the direction of longitudinal extension of the tool is aligned with the midsagittal plane. Preferably, the at least one pointer insertion member of the distal side is arranged at one end or close to one end of the longitudinal extension of the tool. In particular, in longitudinal extension of the tool, the pointer insertion members are positioned in one section of the distal side (called "first section") while no pointer members are present in the reminder of the distal side (called "right section"). In this way, the detection of points which are lying in a plane, in particular the midsagittal plane, and which are not accessible can be replaced by detection of the position of the pointer insertion members which are accessible since they are placed closed to one end (called first end) of the tool (in longitudinal direction), in particular within one half (called first half) of the tool (in the first section). In particular the second section is within the other half of the tool or comprises the other half of the tool (called 2^{nd} half). Thus, the at least one pointer insertion member is (in longitudinal direction) closer to the first end of the tool than to the other end (called second end) of the tool. Preferably, that one of the at least one pointer insertion members which is closest to the second end has at least a distance of 5 cm, 10 cm, 15 cm or 20 cm from the second end, preferably at least 15 cm. In this way, the pointer insertion members do not interfere with fixing devices which are used to fix the anatomical body part in particular during surgery and which contact the right section but do not contact the left section.

Preferably, the plane point detection tool (shortly just called "tool") comprises at least two pointer insertion members in order to detect two points in a plane of the anatomical body part. Preferably, the distance between that one of the two pointer insertion members which is closest to the first end and that one of the pointer insertion member which is closest to the second end is at least 1 cm, 3 cm, 5 cm or 10 cm, preferably at least 3 cm. The greater the distance, the higher is the accuracy of detection of a position, in particular orientation of a plane. Preferably, that one of the at least two pointer insertion members which is closest to the second end is (still) closer to the first end than to the second end. According to a further embodiment, two pointer insertion members have a distance of preferably at least 5 cm or 10 cm or 15 cm (preferably at least 10 cm) and in particular allow a patient fixing device to be positioned in between. In particular in that case, that one of the at least two pointer insertion members which is closest to the second end is closer to the second end than to the first end.

Preferably, the distal side comprises a flat surface section, in particular plane surface section which is closer to the second end than to the first end. The length of these flat surface portion (in longitudinal direction of the tool) is preferably longer than 5 cm, 10 cm, 15 cm or 20 cm. Due to the flat surface section, pressing of a fixing device to an anatomical body part is not obstructed. In particular, the flat surface section adopts at least a part of the second half of the distal side which is closest to the second end.

Preferably, the left section comprises a flat section facing in distal direction. Preferably, the pointer insertion members are projecting from the flat surface section which is closer to the first end than to the second end. In this way, the pointer insertion members are palpable (can be sensed by a member of the medical stuff) due to difference in height of the flat section and the distal end of the prominent pointer insertion member, even through a drape which covers the distal side of the tool. Thus, it is possible to insert a pointer tip into the pointer insertion member although there is a drape between the pointer tip and the tapered recess of the pointer insertion member. In this way, it is possible that the drape can rest on the patient during detection of the points lying in the plane of the anatomical body part (in particular midsagittal plane).

Preferably, the proximal side is constituted to be fitted to a patient. The proximal side of the tool is in particular fitted to a patient by contacting the proximal side with the patient. The contact is in particular along the first direction of longitudinal (length-wise) extension of the tool. Preferably, there are a plurality of contacts, in particular a multiplicity (manifold) of contacts between the patient and the tool in order to achieve the fitting. To this end, the proximal side for instance comprises an adhesive which is constituted to adhere the tool to the patient's body. According to another embodiment, the proximal side comprises a plurality of sucking portions (e.g. sucking cups) which allow to fit (in particular fix) the tool to the body.

Preferably, the plane point detection tool is flexible if flexed in proximal direction. This proximal direction is called third direction. The third direction is in particular normal to the surface of the proximal side. Since the tool is flexible in the third direction, the tool can be attached to a curved surface of the body part. Preferably, the tool is rigid in a second direction. In other words, the tool is preferably not bendable in the second direction. The second direction is preferably perpendicular to the third direction and to the first direction. The second direction is preferably a width-wise direction of the tool while the first direction is preferably a length-wise (longitudinal) direction of the tool. Preferably, the length of the longitudinal extension of the tool is longer than the length of the width-wise extension of the tool. Preferably, the length-wise extension of the tool is longer than three times, five times or ten times of the width-wise extension of the tool. Due to the longer extension of the tool in the lengthwise direction than in the width-wise direction an adaptation of the shape of the tool to the shape of the surface of the body of the patient is possible although the tool is rigid in the widthwise direction. Preferably, the width of the tool in width-wise direction (at least with respect to the second section) is smaller than 5 cm, 3 cm, 2 cm or 1 cm and/or larger than 1 mm, 2 mm or 5 mm. The width of the tool at the first section can be wider than at the second section.

Preferably, the tool is constituted to be fitted to the body at least within a part of the section which is opposite to the second section at the proximal side.

In this way, the tool is attachable to the body at least in a section of the tool which is assumed to be obstructed by the aforementioned fixing device while the fixing device can be brought into contact with the anatomical body part and the second section without interfering of the plane point detection tool. In particular, the fixing device can exert pressure on the anatomical body part via the tool. Preferably, to this end, the thickness of the plane point detection tool is smaller than the length and in particular also smaller than the width of the plane point detection tool. Preferably, the thickness (i.e. the extension of the tool in the third direction) is preferably smaller than a half, in particular smaller than a fifth, in particular smaller than a tenth of the width of the tool. The thickness of the tool (at least with respect to the second section) is preferably smaller than 1 cm, 5 mm, 2 mm or 1 mm. The aforementioned definitions of the thickness of the tool apply in particular for the second section of the tool which is close to the second end and which comprises or consists of a flat surface. The first section which includes at least one pointer insertion member can have a maximum thickness which is larger than the thickness as defined before. In particular, the thickness at the position of the pointer insertion members can be more than 1 mm or 2 mm or 5 mm or 1 cm thicker than the thickness of the second section (in particular due to the height of the pointer insertion member) and/or can be a half or equal to or twice the width of the plane point detection tool.

In the following detailed description, other features of the present invention are disclosed. The features of different embodiments can be combined.
Figure 1 shows a navigation system in accordance with the invention;
Figure 2 shows the use of main and auxiliary points for determining the mid-sagittal plane;
Figure 3 shows a plane point detection tool;
Figures 4 to 6 show situations of use of the plane point detection tool.

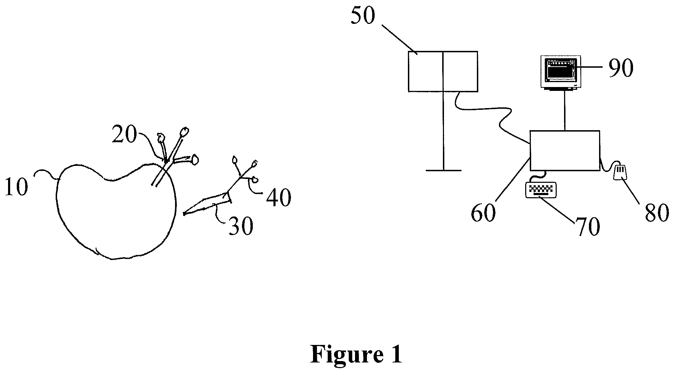

Figure 1 schematically shows a pelvis 10. Attached to the pelvis 10 is a reference star 20. There is a fixed spatial relationship between the reference star 20 and the pelvis 10. A surgeon can contact parts, in particular landmarks, of the pelvis 10 by means of the tip of a pointer 30. Attached to the pointer 30 are markers, in particular another reference star 40. The location of the reference stars 20 and 40 (and the corresponding markers) can be detected by the detection device 50. The process of contacting the pelvis with the pointer and detecting the markers of the pointer is also referred to here as "scanning". The detection device 50 supplies the detection signals to the computer 60. The computer 60 includes a database which stores the relative spatial relationship between the markers of the pointer and the tip of the pointer. In the computer 60, the absolute point data are then determined on the basis of the detection signals received from the detection device 50. The relative point and/or pelvis data can be input or can be stored in the database. Due to this determination, the absolute point data are available and thus provided.

A keyboard 70 and mouse 80 and a monitor 90 are for example connected to the computer 60. The monitor 90 serves to display information to the user such as the results of the processing performed in accordance with a data processing method of the invention and/or indication signals as already mentioned above.

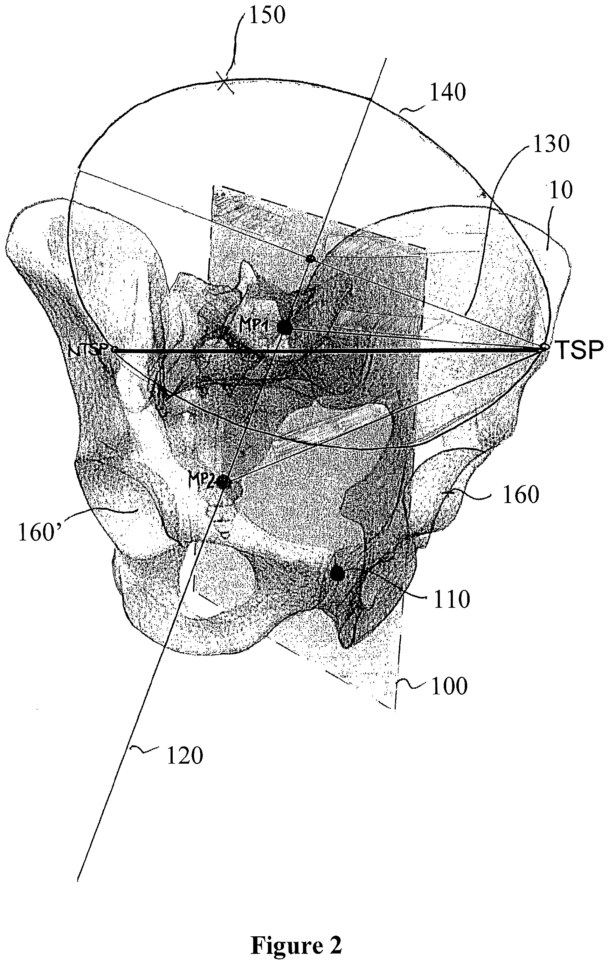

Figure 2 shows the pelvis 10 in more detail. The attached reference star 20 is not shown. Figure 2 also shows a landmark TSP which is the ASIS point. This landmark TSP has been scanned by the pointer 30, and the corresponding position information has been supplied to the computer 60 of the navigation system. The navigation system comprises the detection device 50, the computer 60 and the monitor 90. Two landmarks MP1 and MP2 have also been scanned by the pointer 30. These landmarks MP1 and MP2 lie in the mid-sagittal plane 100. For instance, in the following the points MP1 and MP2 are assumed to be points on the sacrum and lumbar spine. The point TSP is an example of an auxiliary point, and the points MP1 and MP2 are examples of main points. Another main point would be the pubic landmark 110. However, in the following, it is assumed that the absolute main point data describe only the position of MP1 and MP2 and that the absolute auxiliary point data describe only the position of TSP. In the following, it will be described how the position of the main plane (the mid-sagittal plane 100) is determined on the basis of only two main points and only one auxiliary point.

In accordance with one possible procedure, a line 120 is determined by MP1 and MP2. A line 130 is drawn from the point TSP and intersects the line 120 at a right angle. Thus, the line 130 is perpendicular to the line 120. The intersection between the line 120 and the line 130 defines the center of a circle 140. The circle 140 includes the point TSP and another point NTSP. The point NTSP is a virtual auxiliary point (i.e. NTSP has not been scanned). The point NTSP is symmetrical to the point TSP with respect to the mid-sagittal plane 100. In other words, if the point TSP is mirrored in the mid-sagittal plane 100, this would result in the point NTSP. The point NTSP is the solution which is to be found. Relative point data are used to find the point NTSP. The relative point data describe the distance between NTSP and TSP. This distance can for example be derived from an x-ray of the pelvis 10. The point NTSP has to be on the circle 140 and has to fulfill the constraint, i.e. has to have the predetermined distance from the point TSP. There are two possible solutions for these constraints, namely NTSP and the point 150. The point 150 is an incorrect solution. The pelvis data are used to discount the incorrect solution 150 from the two possible solutions. The pelvis data describe in particular anatomical characteristics of the pelvis. These data describe in particular that the point NTSP is more anterior than the point MP1. The point 150, however, is more posterior than MP1. Therefore, this possible solution can be discounted. The solution NTSP is therefore selected, since this point complies with the anatomical characteristic that the solution has to be more anterior than MP1. The constraint that NTSP has to be more anterior than MP1 represents an example of the possible relative positions between MP1 and NTSP. These possible relative positions are constraints described by the pelvis data. The circle 140 is incidentally also perpendicular to the line 120.

In accordance with another possible data processing method, a sphere is constructed which has a radius corresponding to the distance between MP1 and TSP. The center of this sphere is located at the point MP1. A second sphere is also constructed which has a radius corresponding to the distance between MP2 and TSP. The center of the second sphere is located at the point MP2. The intersection between these two spheres is the circle 140 which corresponds to a multitude of possible solutions for the location of the point NTSP. In the same way as mentioned above, this multitude of possible solutions is firstly restricted to the point NTSP and the point 150 by using the constraint which defines the distance between NTSP and TSP. In a subsequent step, the pelvis data are then used to discount the point 150 from the possible solutions, such that the point NTSP remains as the only possible solution.

As mentioned above, NTSP and TSP are symmetrical to each other with respect to the mid-sagittal plane. Therefore, the mid-sagittal plane is defined as being perpendicular to the line which connects NTSP and TSP, hence the mid-sagittal plane is determined.

In accordance with another preferred step, another plane - the auxiliary plane - is determined, with the aim of defining a coordinate system in which the pelvis rests. For this purpose, another auxiliary point 160 is provided. To this end, the landmark corresponding to the auxiliary point 160 is preferably scanned by the pointer 30. The auxiliary plane is then defined as being perpendicular to the mid-sagittal plane 100 and including the two auxiliary points TSP and 160.The auxiliary point 160 is preferably based on the acetabulum. It can for example be the rotational center of the femoral head in the acetabulum or the bottom of the acetabulum (fossa) or the center of the rim of the acetabulum, to name but a few examples. The inventors of the present application have found that there is a reliable and fixed spatial relationship between a standard plane (APP) and the aforementioned auxiliary plane which includes a landmark defined by the acetabulum and the ASIS point as another auxiliary point. The point TSP preferably corresponds to the ASIS point, in which case the auxiliary plane has a defined positional relationship to the anterior pelvic plane (APP) which is often used as a reference for defining the orientation of the acetabulum. This standard plane is also perpendicular to the mid-sagittal plane and has an angle of about 40° with respect to the aforementioned plane which includes the ASIS point and the acetabulum landmark point. This auxiliary plane is also referred to here as the spinae joint center plane (SJCP).

In the manner described above, at least two planes are defined which allow a coordinate system to detected defined. The planes can for example be the mid-sagittal plane and the auxiliary plane (in particular the SJCP) and/or can be the mid-sagittal plane and the APP.

In the following, the data processing method is described for the scenario in which there is only one main point on the mid-sagittal plane. In this case, the relative point data preferably comprise two constraints or more specifically two scalar values. One scalar value describes the distance between the point NTSP and the point TSP. The other scalar value describes the distance between the acetabular or auxiliary point 160 and the symmetrical acetabular or auxiliary point 160'. This distance is in particular the aforementioned inter-fossa distance. In other words, the symmetrical acetabulum 160' is symmetrical with respect to the mid-sagittal plane, i.e. the point 160' is a mirrored point of the acetabular point 160.

The two scalar values, i.e. the two distances, are in particular known from x-ray images. The distance between the point TSP and the point 160 is also known due to the absolute auxiliary point data. In a subsequent step, a line can be constructed on the basis of the TSP point and the point 160. This line crosses the mid-sagittal plane 100. The distance between the point 160 and the mid-sagittal plane 100 along this line can be calculated on the basis of geometric laws by using the aforementioned two distances between the point TSP and the point NTSP and between the point 160 and the point 160'. Thus, the position at which the line which includes the point TSP and the point 160 crosses the mid-sagittal plane can be calculated. Thus, a second point on the mid-sagittal plane is known. The procedure already known from the above description can then be applied in order to determine the position of the point NTSP or the point 160'. If these positions are known, then the mid-sagittal plane is defined as being perpendicular to the line connecting the point NTSP and the point TSP and/or perpendicular to the line connecting the point 160' and the point 160. The SJCP or the APP can also then be calculated in the manner described above.

The above-mentioned inter-teardrop distance can be measured in an anterior/posterior x-ray image of the pelvis. Within an extensive analysis of CT data sets it had be found by the inventors that the inter-teardrop distance as well as the inter-fossa distance are almost constant across various populations. Between males and females there are significant differences. Thus, a gender-specific value for the distances is preferably used as relative point data. For fine-tuning purposes, the values could also be adjusted to certain populations (e.g. Asian, European, US). In particular, a fixed value for the distance can be assumed for a specific population in order to provide the relative point data.

The inter-teardrop and inter-fossa distances are in particular defined as described in the following. The teardrop figure is a well-known structure which can be identified in anterior/posterior x-rays of the pelvis. Within the mentioned CT data analysis, artificially generated x-ray images (i.e. digitally reconstructed radiographs-DRRs) were used to reproduce the teardrop figure for evaluation purposes. The teardrop figure depicts a structure close to the medial wall of the acetabulum and can be determined on each side of the pelvis. In particular the medial-lateral position of the structure can be determined very well. The inter-teardrop distance is a distance between the teardrop figures on a medial-lateral line through the pelvis.

The fossa is a region within the acetabulum which is deeper than the rest of the approximately spherical structure of the acetabulum. It basically lies on the medial side of the acetabulum. The inter-fossa distance is the distance between the fossa regions on both sides of the pelvis in a medial/lateral direction. The fossa region can be easily identified intra-operatively, e.g. palpated with a pointer device and acquired with a navigation system. For evaluation purposes, the fossa region can be identified directly in the CT datasets.

The fossa region is an anatomical structure which significantly influences the position of the teardrop figure (see also Bowerman JW, Sena JM, Chang R: The teardrop shadow of the pelvis; anatomy and clinical significance. Radiology 1982 Jun; 143(3):659-62). Thus, both distance values (inter-fossa distance and inter-teardrop distance) are strongly correlated. For practical purposes, the inter-teardrop and/or the inter-fossa distance can be used.

Typical values for the distances are 112 mm (inter-teardrop) and 116 mm (inter-fossa) for males. For females, the standard values are 121 mm (inter-teardrop) and 125 mm (inter-fossa). For Asian people, the values are a bit lower (110 mm inter-teardrop and 113 mm inter-fossa for males, 117 mm inter-teardrop and 119 mm inter-fossa for males). Additionally, other procedures to determine orientations of the pelvis or other planning parameters can be based on the proposed distances as well. In particular the orientation according to rotations around a cranial/caudal axis can be determined. In particular information about the anteversion of the acetabulum/cup implant e.g. for THR surgeries can be determined.

Using the SJCP to define a coordinate system can be advantageous, since the APP might be less reliably determined by scanning, i.e. using a pointer to detect the landmarks on the APP. The pubic landmark 110 in particular can introduce uncertainties into the method due to differing amounts of fat and other soft tissue above the pubic area. These tissues can prevent the position of the point 110 from being accurately detected by means of a pointer.

The coordinate systems defined in accordance with the method of the present invention can be used for registration and also for other tasks within planning and navigation steps. The method of the present invention (i.e. the data processing method) is in particular used for planning surgery and for computer-assisted navigation. Because of the reliable and consistent relationship between a coordinate system defined by the mid-sagittal plane and SJCP and standard coordinate systems, all information can be transferred back and forth between the coordinate systems.

In accordance with another embodiment, one main point is scanned by the pointer on the mid-sagittal plane. In other words, one main point is provided to the data processing method. One additional (first) point on a structure, in particular a prominent structure, of the pelvis is determined as an auxiliary point (symmetrical auxiliary point). An example of the prominent structure is the posterior aspect of the iliac crest. The data processing method of the present invention, performed in particular on the navigation system of the invention, then assists the user in finding another (second) auxiliary point by issuing the indication signal mentioned above. This second point is symmetrical to the first point with respect to the mid-sagittal plane. For this purpose, the navigation system shows additional information (indication signals) such as distance values or angles. The rationale behind this approach is that the user is assisted in finding the second point which has the same relationship (for example distance/angle) to the mid-sagittal plane as the first point. The user can thus proceed along the prominent structure until the system informs the surgeon that the desired relationship has been found. In general, this approach can be used to find new reference points (new auxiliary points) on an anatomical object from given points and the specific relationships (geometric features/constraints) between the given point and the points to be determined. The navigation system according to the invention assists in finding the new points (new auxiliary points) by providing information concerning the geometric features/constraints. The user can thus find these new auxiliary points by locating them on a prominent structure. This prominent structure can be close to the mid-sagittal plane.

The above-mentioned embodiment can in particular be used in the case of lateral pelvic registration. The embodiment can be used to find a second point which lies symmetrical to the first point with respect to the mid-sagittal plane. The line between these two points represents the medial-lateral direction. This can be used as an additional constraint. It provides not only a one-dimensional but a two-dimensional constraint. Thus, only one point on the mid-sagittal plane is required in this case. In the same way as in the previous embodiments, additional information (relative point data and/or pelvis data) can be used to make the registration more robust.

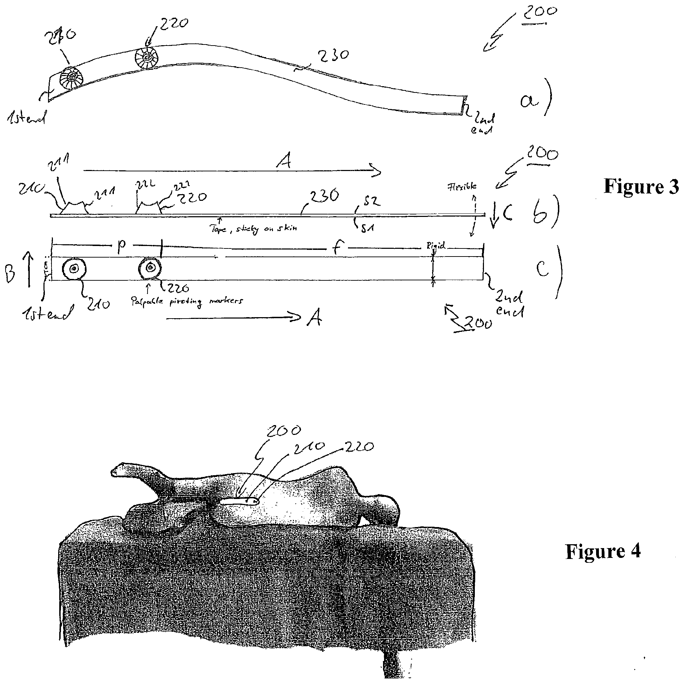

Figure 3a, 3b and 3c show an embodiment of a plane point detection tool. Figure 3a is a perspective view of the embodiment. Figure 3b is a side view of the embodiment. Figure 3c is a top view of the embodiment.

Figure 3c shows the embodiment of the plane point detection tool from the bird's eye view (top view). The arrow A shows the direction of longitudinal (length-wise) extension of the tool. The arrow B shows the direction of width-wise extension of the tool. The width-wise extension of the tool is for example 1 cm and preferably has an extension larger than 3 mm and/or smaller than 4 cm. This upper end and/or lower limit applies in particular for the section "f" (second section) which is the section on the right side in Figure 3c and which extends from the second end of the tool over a major part of the tool towards but not up to the first end. The remaining part of the tool is called section "p" (first section). This section p extends from the first end of the tool at least until it includes that one of the pointer insertion elements which is closest to the second end. According to an embodiment, the section p (first section) and section f (second section) are adjoining to each other. Section f includes in particular a major part of the tool (in longitudinal direction of the tool). In particular the section f is preferably constituted to be fitted to a patient. To this end, preferably the distal side and/or the proximal side of the tool is flat, in particular plane. Figure 3c shows the distal side of the tool 200. In particular, two pointer insertion tools 210 and 220 are prominent, in particular prominent from a flat portion 230 which preferably extends from the first end to the second end. The flat portion 230 comprises in particular opposite flat surfaces S1 and S2 (see Figure 3b). The surface S2 faces towards the distal side and the surface S1 faces towards the proximal side. The flat portion 230 has preferably a tape like shape. In particular the surface S1 and S2 have a rectangular shape. Preferably the extension of the flat portion in the direction A is longer than the extension in the width-wise direction B for more than a factor 2, in particular 3, preferably 5. The flat portion 230 is preferably rigid in the direction B while it can be bent in a direction C. That is, the flat portion 230 is preferably flexible in the direction C. That is, in the side view from a direction as shown in Figure 3b, the tool 200 can adopt a curved or undulated shape as is indicated by the perspective view shown in Figure 3a. Preferably, the tool is rigid in the direction B, i.e. cannot be bent. Rigid means in particular that the tool cannot be bent (e.g. by at least 1 cm in the direction B at the second end) by a force applyable by human being which is in particular a force of more than 10 N and less than 1000 N. The force is applied on one end of the tool (e.g. second end) while the other end (e.g. first end) is fixed. Preferably, such a force which results in a bending (e.g. by at least 1 cm in the direction (e.g. at the second end) is smaller than 1000 N, in particular smaller than 100 N or 10 N if the force is applied in the direction C (at one end, e.g. 2^{nd} end of the tool while fixing the other end, e.g. first end of the tool).

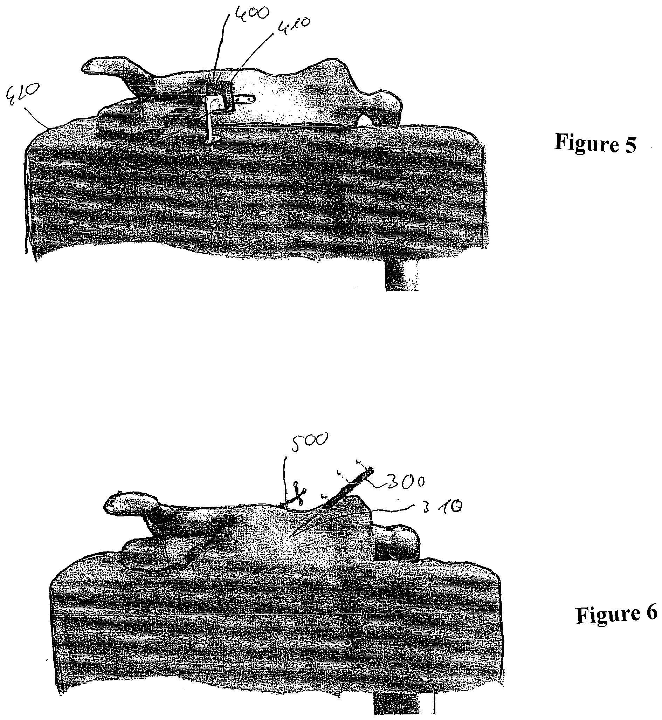

Preferably, the pointer insertion elements 210 and 220 are attached to the flat portion, for instance by gluing or by means of a fixing element (for instance a screw or a rivet). The pointer insertion tools have preferably prominent outside walls 211 and 222 (see Figure 3b) which are preferably projecting from the flat surface S2 into the distal direction. Inside the walls there is preferably a recess, in particular pointed recess which allows to insert the tip 310 of a pointer 300 (see Figure 6). The recess has in particular conical shape and is preferably tapered from the distal end of the pointer insertion elements towards the second surface (i.e. in proximal direction).

Besides the rectangular shape shown in Figure 3, the tool can for instance also have oblong or elliptical shape.

Figure 4 shows the tool 200 in use. The tool 200 is attached to the back of a patient is aligned with the midsagittal plane of the pelvis. The alignment is possible by sensing the spinus process of the sacrum. In particular, the tool 200 is attached by means of an adhering layer which comprises the surface S1 of the tool 200. In this way, the tool 200 can be attached to the skin as shown in Figure 4. In other words the tool 200 has preferably the properties of a sticky tape, in particular is constituted as a sticky tape. Since the tool is flexible in the direction C, the tool can be smoothly (snugly) fitted to the curved surface of the patient's skin. Since the tool is small in width-wise direction, the rigid property of the tool in the width-wise direction does not hinder the attachment of the tool to the curved skin. Additionally, the rigid property assures that the pointer insertion elements 210 and 220 are aligned in the same direction as the longitudinal extension of the section f of the tool. Thus, if the longitudinal extension of the section f is aligned with the midsagittal plane also a virtual line which connects the at least two pointers 210 and 220 is aligned with the midsagittal plane. Thus the at least one pointer insertion element (210 and 220) are positioned in the midsagittal plane.

Figure 5 shows the attachment of a patient fixing device 400 comprising a cushion 410 and a support structure 420 for the cushion which is fixed to the couch on which the patient is lying. The cushion 410 is in contact with the back skin of the patient and the area of the pelvis and thus obstructs at least a part of the section f of the tool 200. The fixing device is also called posterior support.

Thereafter, as shown in Figure 6 the patient can be draped as usual. In particular a marker device (reference star 500) can be fixed to the pelvis of the patient. After fixing the reference star 500 it is of advantage to teach the navigation system the position of the plane (in particular midsagittal plane) relative to the reference star 500 in order to help the surgeon during surgery by means of computer assisted surgery (navigation). Without the tool 200, the problem would be here that the projecting parts of the sacrum cannot be sensed (palpated) or are difficult to be sensed by the surgeon or any other member of the medical staff. However, due to the tool 200, the pointer insertion elements 210 and 220 can be sensed (palpated) even through the drape since the pointer insertion tools are prominent and their recesses are sensible. In particular, the dimension of the opening of the recess in the pointer insertion member is preferably larger than 1 mm, 3 mm or 5 mm and in particular smaller than 2 cm or 1 cm. Therefore, the operator (surgeon or any other member of the medical staff) is able to insert the tip 310 of the pointer 300 into the recess of the pointer insertion member 210 and 220. In this way, the operator can teach to the navigation system the positions of the tips of the recesses 210 and 220 which are positioned in the midsagittal plane. In particular the detection of the position of the pointer (by detecting the markers of the pointer) allows to determine the position of the tip of the pointer (due to data describing the relative position between the markers of the pointer and the tip of the pointer) and to thus generate absolute main point data.

## Claims

1. A data processing method for determining the position of a mid-sagittal plane of a pelvis, performed by a computer and comprising the steps of:
• providing absolute auxiliary point data which describe the position of at least one actual auxiliary point of the pelvis relative to a marker device attached to the pelvis, the at least one actual auxiliary point being outside the mid-sagittal plane but on the same side of the mid-sagittal plane, and representing an anatomical landmark of the pelvis;
• providing relative point data which constrain the possible positions of the mid-sagittal plane relative to the at least one actual auxiliary point;
• providing absolute main point data which describe the position of one or two actual main points of the pelvis relative to the marker device attached to the pelvis, said one or two actual main points lying in the mid-sagittal plane and representing an anatomical landmark on the pelvis
• calculating a position of the mid-sagittal plane relative to the marker device, wherein the calculation uses the relative point data and auxiliary point data as well as the provided absolute main point data;
wherein,
a) if the absolute main point data describe the position of two actual main points,
i) then the relative point data describe at least one constraint, and the calculating step uses the at least one constraint as well as the position of at least one actual auxiliary point and the positions of the two actual main points; and
ii) then the calculating step comprises:
determining at least one virtual auxiliary point by using the relative point data, the absolute main point data, and the absolute auxiliary point data, the relative point data constraining the possible positions by describing a shortest distance between the mid-sagittal plane and a particular auxiliary point of the at least one actual auxiliary point and/or by describing a distance between the virtual auxiliary point and the particular auxiliary point, the virtual auxiliary point being symmetrical to the particular auxiliary point with regard to the mid-sagittal plane and representing an anatomical landmark on the pelvis; or
b) if the absolute main point data describe the position of one actual main point,
i) then the relative point data describe at least two constraints, and the calculating step uses the at least two constraints as well as the positions of at least two of the actual auxiliary points and the position of the one actual main point, the two actual auxiliary points being on the same side of the mid-sagittal plane, the relative point data containing the possible position of the mid-sagittal plane relative to the actual auxiliary points by describing a positional relationship defined by shortest distances between the actual auxiliary points and the mid-sagittal plane, and
ii) then the calculating step comprises:
determining a position of a virtual main point based on the relative point data, by using two of the at least two actual auxiliary points, the two actual auxiliary points defining a line which crosses the mid-sagittal plane; and
determining, based on the position of the virtual main point and the one actual main point, at least one virtual auxiliary point by using the relative point data which describe a distance between the mid-sagittal plane and a particular auxiliary point of the at least two actual auxiliary points and/or between the virtual auxiliary point and the particular auxiliary point, the virtual auxiliary point being symmetrical to the particular auxiliary point with regard to the mid-sagittal plane.
- wherein the position of the mid-sagittal plane is determined on the basis of the particular auxiliary point and the symmetrical virtual auxiliary point in that the mid-sagittal plane is defined to be perpendicular to a line connecting the particular auxiliary point and the symmetrical virtual auxiliary point.

2. The data processing method according to any one of the preceding claims, wherein the position of the virtual auxiliary point is symmetrical to the position of the particular auxiliary point with respect to the mid-sagittal plane.

3. The data processing method according to any one of the preceding claims, further comprising the steps of:
• providing data called pelvis data which constrain the possible relative positions between landmarks of the pelvis and/or between the landmarks and the mid-sagittal plane;
• providing data called landmark data which respectively correlate at least some of the actual and/or virtual main points and/or auxiliary points with at least some of the landmarks of the pelvis; and
• if more than one solution for calculating the position of the mid-sagittal plane is possible, selecting one of the possible solutions for the position of the mid-sagittal plane on the basis of the landmark data and pelvis data.

4. A data processing method which includes the data processing method of any one of the preceding claims and which is a method for determining the position of the mid-sagittal plane of the pelvis and of a spinae joint center plane of the pelvis, the spinae joint center plane being referred to as SJCP, wherein in order to determine the mid-sagittal plane, the data processing method of any one of the preceding claims is performed, and in order to determine the SJCP, absolute auxiliary point data describe the position of at least two of the auxiliary points of the pelvis relative to the marker device, the at least two auxiliary points being outside the mid-sagittal plane; further comprising the step of providing relative auxiliary plane data which describe a predetermined positional relationship between the SJCP and the mid-sagittal plane;
wherein the position of the SJCP is determined by assuming that the at least two auxiliary points lie in the SJCP, and is determined on the basis of the relative auxiliary plane data.

5. A data processing method which includes the data processing method of the preceding claim for determining the position of the mid-sagittal plane and of the SJCP and which is additionally a method for determining the position of an anterior pelvic plane, further comprising the steps of:
providing relative standard plane data which describe the expected positional relationship between the SJCP and the anterior pelvic plane; and
determining the position of the anterior pelvic plane on the basis of the position of the SJCP and the relative standard plane data.

6. The data processing method according to any one of the preceding claims, wherein: one of the landmarks represented by one of the auxiliary points is the sinistral or dextral anterior superior iliac spine landmark; and/or
the relative standard plane data describe the angle between the anterior pelvic plane and the SJCP; and/or
the anterior pelvic plane and the SJCP intersect each other along a line connecting the sinistral and the dextral anterior superior iliac spine landmarks of the pelvis; and/or
another auxiliary point represents a landmark defined by the acetabulum or a part of the acetabulum or the fossa of the acetabulum or another point of the acetabulum; and/or
the relative point data describe a constraint for an inter-fossa distance or an inter-teardrop distance of the pelvis.

7. A data processing method for determining additional points called additional auxiliary points which can be used for determining the position of the mid-sagittal plane, according to any one of the preceding claims, comprising the steps of:
providing at least one auxiliary point;
providing relative auxiliary point data which describe at least one constraint which limits the possible positions for an additional auxiliary point, the additional auxiliary point being symmetrical to one of the one or more auxiliary points with respect to the mid-sagittal plane; and
providing candidate point data in steps, wherein in each step, the position of a new candidate point is described by the candidate point data, wherein in the current step, the following steps are performed:
a) providing new data called new candidate point data which describe a relative position of a new candidate point with respect to the marker device, the new candidate point being a new candidate for a position of the additional auxiliary point;
b) checking whether the positions of the new candidate point comply with the at least one constraint described by the relative auxiliary point data;
c) repeating steps a) and b) if the position of the new candidate point does not comply with the at least one constraint, or accepting the new candidate point as an additional auxiliary point if the position of the candidate point does comply with the at least one constraint.

8. A program which, when it is running on a computer or is loaded onto a computer, causes the computer to perform the method according to any one of the preceding claims.

9. A program storage medium on which the program of claim 8 is stored.

10. A computer on which the program of claim 8 is running or in the memory of which the program of claim 7 is loaded.

11. A navigation system for computer-assisted surgery, comprising:
the computer of the claim 10;
a detection device for detecting the position of the main and auxiliary points and for generating detection signals which represent the position of the main and auxiliary points and for supplying the detection signals to the computer of claim 9, the computer being designed to process the absolute main point data and the absolute auxiliary point data, and the relative point data in accordance with any one of the method claims 1 to 6; and
a user interface for receiving data from the computer in order to provide information to the user, the computer being designed to calculate the data in accordance with the data processing method according to any one of the preceding method claims.

12. A determination method for determining the position of a mid-sagittal plane of a pelvis, comprising the steps of:
generating detection signals by detecting a pointer, said pointer contacting a pelvis or a tool, called main point detection tool;
determining, on the basis of the detection signals, the absolute main point data and absolute auxiliary point data as mentioned in any one of the method claims 1 to 6, and then performing any one of the preceding method claims 1 to 6 on the basis of the determined absolute main point data and absolute auxiliary point data.

13. The navigation system of claim 11 further comprising a tool, called plane point detection tool, for detecting at least one actual main point, the plane point detection tool comprising:
a proximal side constituted to be fitted to a patient by contacting the patient along a first direction of length-wise extension of the tool, the proximal side is constituted to be able to be aligned with a plane of an pelvis along the first direction of length-wise extension, the direction in which the proximal side faces and along which the tool is flexibly bendable for bringing the tool into contact with the patient being a third direction of extension of the tool;
a distal side which comprises at least one pointer insertion member comprising a tapered recess for inserting the tip of a pointer, the at least one pointer insertion member being prominently positioned in a first section of the distal side which is, in the direction of length-wise extension, closer to a first end of the plane point detection tool than to a second end;
wherein the distal side further comprises a second section having a flat surface and being closer to the second end than to the first end;
both the proximal side and distal side extending in the first direction and in a second direction of extension, the second direction being traverse to the first direction,
the tool being flexibly bendable in the third direction while being rigid in the second direction, the second direction being a direction of width-wise extension of the tool, the width-wise extension of the tool being smaller than the length-wise extension.

## Patentansprüche

1. Datenverarbeitungsverfahren zum Bestimmen der Position einer Medianebene eines Beckens, das von einem Computer durchgeführt wird und die folgenden Schritte umfasst:
• Bereitstellen von absoluten Hilfspunktdaten, die die Position von mindestens einem tatsächlichen Hilfspunkt des Beckens relativ zu einer am Becken befestigten Markierungsvorrichtung beschreiben, wobei der mindestens eine tatsächliche Hilfspunkt außerhalb der Medianebene, aber auf derselben Seite der Medianebene liegt und eine anatomische Landmarke des Beckens repräsentiert;
• Bereitstellen von relativen Punktdaten, die die möglichen Positionen der Medianebene relativ zu dem mindestens einen tatsächlichen Hilfspunkt einschränken;
• Bereitstellen von absoluten Hauptpunktdaten, die die Position von einem oder zwei tatsächlichen Hauptpunkten des Beckens relativ zu der am Becken befestigten Markierungsvorrichtung beschreiben, wobei der eine oder die zwei tatsächlichen Hauptpunkte auf der Medianebene liegen und eine anatomische Landmarke des Beckens repräsentieren
• Berechnen einer Position der Medianebene relativ zur Markierungsvorrichtung, wobei die Berechnung die relativen Punktdaten und Hilfspunktdaten sowie die bereitgestellten absoluten Hauptpunktdaten verwendet;
wobei,
a) wenn die absoluten Hauptpunktdaten die Position von zwei tatsächlichen Hauptpunkten beschreiben,
i) dann beschreiben die relativen Punktdaten mindestens eine Einschränkung und der Berechnungsschritt verwendet die mindestens eine Einschränkung sowie die Position von mindestens einem tatsächlichen Hilfspunkt und die Positionen der zwei tatsächlichen Hauptpunkte; und
ii) der Berechnungsschritt dann Folgendes umfasst:
Bestimmen von mindestens einem virtuellen Hilfspunkt durch Verwenden der relativen Punktdaten, der absoluten Hauptpunktdaten und der absoluten Hilfspunktdaten, wobei die relativen Punktdaten die möglichen Positionen durch Beschreiben eines kürzesten Abstands zwischen der Medianebene und einem bestimmten Hilfspunkt des mindestens einen tatsächlichen Hilfspunktes und/oder durch Beschreiben eines Abstands zwischen dem virtuellen Hilfspunkt und dem bestimmten Hilfspunkt einschränkt, wobei der virtuelle Hilfspunkt mit Bezug auf die Medianebene zum bestimmten Hilfspunkt symmetrisch ist und eine anatomische Landmarke am Becken repräsentiert; oder
b) wenn die absoluten Hauptpunktdaten die Position von einem tatsächlichen Hauptpunkt beschreiben,
i) dann beschreiben die relativen Punktdaten mindestens zwei Einschränkungen und der Berechnungsschritt verwendet die mindestens zwei Einschränkungen sowie die Positionen von mindestens zwei der tatsächlichen Hilfspunkte und die Position des einen tatsächlichen Hauptpunktes, wobei sich die zwei tatsächlichen Hilfspunkte auf derselben Seite der Medianebene befinden, wobei die relativen Punktdaten durch Beschreiben einer Positionsbeziehung, die durch die kürzesten Abstände zwischen den tatsächlichen Hilfspunkten und der Medianebene definiert ist, die mögliche Position der Medianebene relativ zu den tatsächlichen Hilfspunkten enthalten, und
ii) der Berechnungsschritt dann Folgendes umfasst:
Bestimmen einer Position eines virtuellen Hauptpunktes auf Basis der relativen Punktdaten durch Verwenden von zwei der mindestens zwei tatsächlichen Hilfspunkte, wobei die zwei tatsächlichen Hilfspunkte eine Linie definieren, die die Medianebene kreuzt; und
Bestimmen auf Basis der Position des virtuellen Hauptpunktes und des einen tatsächlichen Hauptpunktes von mindestens einem virtuellen Hilfspunkt durch Verwenden der relativen Punktdaten, die einen Abstand zwischen der Medianebene und einem bestimmten Hilfspunkt der mindestens zwei tatsächlichen Hilfspunkte und/oder zwischen dem virtuellen Hilfspunkt und dem bestimmten Hilfspunkt beschreiben, wobei der virtuelle Hilfspunkt mit Bezug auf die Medianebene zum bestimmten Hilfspunkt symmetrisch ist.
- wobei die Position der Medianebene auf Basis des bestimmten Hilfspunktes und des symmetrischen virtuellen Hilfspunktes bestimmt wird, darin, dass die Medianebene als senkrecht zu einer Linie, die den besonderen Hilfspunkt und den symmetrischen virtuellen Hilfspunkt verbindet, definiert ist.

2. Datenverarbeitungsverfahren nach einem der vorhergehenden Ansprüche, wobei die Position des virtuellen Hilfspunktes mit Bezug auf die Medianebene symmetrisch zu der Position des besonderen Hilfspunktes ist.

3. Datenverarbeitungsverfahren nach einem der vorhergehenden Ansprüche, das ferner die folgenden Schritte umfasst:
• Bereitstellen von Daten, die als Beckendaten bezeichnet werden und die möglichen relativen Positionen zwischen Landmarken des Beckens und/oder zwischen den Landmarken und der Medianebene einschränken;
• Bereitstellen von Daten, die als Landmarkendaten bezeichnet werden und jeweils mindestens einige der tatsächlichen und/oder der virtuellen Hauptpunkte und/oder Hilfspunkte mit mindestens einigen der Landmarken des Beckens korrelieren; und
• wenn mehr als eine Lösung zum Berechnen der Position der Medianebene möglich ist, Auswählen von einer der möglichen Lösungen für die Position der Medianebene auf Basis der Landmarkendaten und der Beckendaten.

4. Datenverarbeitungsverfahren, das das Datenverarbeitungsverfahren von einem der vorhergehenden Ansprüche beinhaltet und ein Verfahren zum Bestimmen der Position der Medianebene des Beckens und einer Wirbelgelenksmittelebene des Beckens ist, wobei die Wirbelgelenksmittelebene als SJCP bezeichnet wird, wodurch zum Bestimmen der Medianebene das Datenverarbeitungsverfahren von einem der vorhergehenden Ansprüche durchgeführt wird und zum Bestimmen der SJCP absolute Hilfspunktdaten die Position von mindestens zwei der Hilfspunkte des Beckens relativ zur Markierungsvorrichtung beschreiben, wobei die mindestens zwei Hilfspunkte außerhalb der Medianebene liegen; ferner den Schritt des Bereitstellens von relativen Hilfsebenendaten umfassend, die eine vorbestimmte Positionsbeziehung zwischen der SJCP und der Medianebene beschreiben;
wobei die Position der SJCP durch Annehmen, dass die mindestens zwei Hilfspunkte in der SJCP liegen, bestimmt wird und auf Basis der relativen Hilfsebenendaten bestimmt wird.

5. Datenverarbeitungsverfahren, das das Datenverarbeitungsverfahren vom vorhergehenden Anspruch zum Bestimmen der Position der Medianebene und der SJCP beinhaltet und das zusätzlich ein Verfahren zum Bestimmen der Position einer vorderen Beckenebene ist und ferner die folgenden Schritte umfasst:
Bereitstellen von relativen Standardebenendaten, die die erwartete Positionsbeziehung zwischen der SJCP und der vorderen Beckenebene beschreiben; und
Bestimmen der Position der vorderen Beckenebene auf Basis der Position der SJCP und der relativen Standardebenendaten.

6. Datenverarbeitungsverfahren nach einem der vorhergehenden Ansprüche, wobei: eine der Landmarken, die durch einen der Hilfspunkte repräsentiert ist, die Landmarke des sinistralen oder dextralen vorderen oberen Darmbeinstachels ist; und/oder
die relativen Standardebenendaten den Winkel zwischen der vorderen Beckenebene und der SJCP beschreiben; und/oder
die vordere Beckenebene und die SJCP sich entlang einer Linie überschneiden, die die Landmarken des sinistralen und des dextralen vorderen oberen Darmbeinstachels des Beckens verbindet; und/oder
ein anderer Hilfspunkt eine Landmarke repräsentiert, die durch die Hüftpfanne oder einen Teil der Hüftpfanne oder die Grube der Hüftpfanne oder einen anderen Punkt der Hüftpfanne definiert ist; und/oder
die relativen Punktdaten eine Einschränkung für einen Inter-Gruben-Abstand oder einen Inter-Tränen-Abstand des Beckens beschreiben.

7. Datenverarbeitungsverfahren zum Bestimmen von zusätzlichen Punkten, die als zusätzliche Hilfspunkte bezeichnet werden und zum Bestimmen der Position der Medianebene verwendet werden können, nach einem der vorhergehenden Ansprüche, das die folgenden Schritte umfasst:
Bereitstellen von mindestens einem Hilfspunkt;
Bereitstellen von relativen zusätzlichen Punktdaten, die mindestens eine Einschränkung beschreiben, die die möglichen Positionen für einen zusätzlichen Hilfspunkt begrenzt, wobei der zusätzliche Hilfspunkt mit Bezug auf die Medianebene zu einem des einen oder der mehreren Hilfspunkte symmetrisch ist; und
Bereitstellen von Punktkandidatendaten in Schritten, wobei bei jedem Schritt die Position eines neuen Punktkandidaten von den Punktkandidatendaten beschrieben wird, wobei beim aktuellen Schritt die folgenden Schritte durchgeführt werden:
a) Bereitstellen von neuen Daten, die als neue Punktkandidatendaten bezeichnet werden und eine relative Position eines neuen Punktkandidaten mit Bezug auf die Markierungsvorrichtung beschreiben, wobei der neue Punktkandidat ein neuer Kandidat für eine Position des zusätzlichen Hilfspunktes ist;
b) Prüfen, ob die Positionen des neuen Punktkandidaten mit der mindestens einen Einschränkung, die von den relativen Hilfspunktdaten beschrieben wird, konform sind;
c) Wiederholen der Schritte a) und b), wenn die Position des neuen Punktkandidaten nicht mit der mindestens einen Einschränkung konform ist, oder Akzeptieren des neuen Punktkandidaten als einen zusätzlichen Hilfspunkt, wenn die Position des Punktkandidaten mit der mindestens einen Einschränkung konform ist.

8. Programm, das, wenn es auf einem Computer ausgeführt wird oder in einen Computer geladen wird, den Computer veranlasst, das Verfahren nach einem der vorhergehenden Ansprüche durchzuführen.

9. Programmspeichermedium, auf dem das Programm von Anspruch 8 gespeichert ist.

10. Computer, auf dem das Programm von Anspruch 8 ausgeführt wird oder in dessen Speicher das Programm von Anspruch 7 geladen wird.

11. Navigationssystem für computergestützte Chirurgie, das Folgendes umfasst:
den Computer von Anspruch 10;
eine Detektionsvorrichtung zum Detektieren der Position der Haupt- und der Hilfspunkte und zum Erzeugen von Detektionssignalen, die die Position der Haupt- und der Hilfspunkte repräsentieren, und zum Liefern der Detektionssignale an den Computer von Anspruch 9, wobei der Computer ausgestaltet ist, um die absoluten Hauptpunktdaten und die absoluten Hilfspunktdaten und die relativen Punktdaten nach einem der Verfahrensansprüche 1 bis 6 zu verarbeiten; und
eine Benutzerschnittstelle zum Empfangen von Daten vom Computer, um dem Benutzer Informationen bereitzustellen, wobei der Computer ausgestaltet ist, um die Daten gemäß dem Datenverarbeitungsverfahren nach einem der vorhergehenden Verfahrensansprüche zu berechnen.

12. Bestimmungsverfahren zum Bestimmen der Position einer Medianebene eines Beckens, das die folgenden Schritte umfasst:
Erzeugen von Detektionssignalen durch Detektieren eines Zeigers, wobei der Zeiger ein Becken oder ein Werkzeug, das als Hauptpunktdetektionswerkzeug bezeichnet wird, berührt;
Bestimmen der absoluten Hauptpunktdaten und der absoluten Hilfspunktdaten auf Basis der Detektionssignale, wie in einem der Verfahrensansprüche 1 bis 6 erwähnt, und dann Durchführen von einem der vorhergehenden Verfahrensansprüche 1 bis 6 auf Basis der bestimmten absoluten Hauptpunktdaten und absoluten Hilfspunktdaten.

13. Navigationssystem von Anspruch 11, das ferner ein Werkzeug, das als Ebenenpunktdetektionswerkzeug bezeichnet wird, zum Detektieren von mindestens einem tatsächlichen Hauptpunkt umfasst, wobei das Ebenenpunktdetektionswerkzeug Folgendes umfasst:
eine proximale Seite, die konstituiert ist, durch Kontaktieren eines Patienten entlang einer ersten Richtung einer längenmäßigen Erstreckung des Werkzeugs an den Patienten angelegt zu werden, wobei die proximale Seite konstituiert ist, entlang der ersten Richtung der längenmäßigen Erstreckung auf eine Ebene eines Beckens ausgerichtet werden zu können, wobei die Richtung, der die proximale Seite zugewandt ist und entlang der das Werkzeug flexibel biegbar ist, um das Werkzeug mit dem Patienten in Kontakt zu bringen, eine dritte Richtung der Erstreckung des Werkzeugs ist;
eine distale Seite, die mindestens ein Zeigereinsatzelement umfasst, das eine konische Ausnehmung zum Einsetzen der Spitze eines Zeigers umfasst, wobei das mindestens eine Zeigereinsatzelement in einem ersten Bereich der distalen Seite, die in der Richtung der längenmäßigen Erstreckung einem ersten Ende des Ebenenpunktdetektionswerkzeugs näher ist als einem zweiten Ende, prominent positioniert ist;
wobei die distale Seite ferner einen zweiten Bereich umfasst, der eine ebene Fläche aufweist und dem zweiten Ende näher ist als dem ersten Ende;
wobei sich sowohl die proximale Seite als auch die distale Seite in die erste Richtung und in eine zweite Richtung der Erstreckung erstrecken, wobei die zweite Richtung quer zur ersten Richtung verläuft,
wobei das Werkzeug in die dritte Richtung flexibel biegbar ist, während es in der zweiten Richtung steif ist, wobei die zweite Richtung eine Richtung der breitenmäßigen Erstreckung des Werkzeugs ist, wobei die breitenmäßige Erstreckung des Werkzeugs kleiner ist als die längenmäßige Erstreckung.

## Revendications

1. Procédé de traitement de données pour déterminer la position d'un plan médio-sagittal d'un pelvis, réalisé par un ordinateur et comprenant les étapes de :
• la fourniture de données de point auxiliaire absolues qui décrivent la position d'au moins un point auxiliaire réel du pelvis par rapport à un dispositif marqueur attaché au pelvis, l'au moins un point auxiliaire réel étant à l'extérieur du plan médio-sagittal mais sur le même côté du plan médio-sagittal, et représentant un repère anatomique du pelvis ;
• la fourniture de données de point relatives qui limitent les positions possibles du plan médio-sagittal par rapport à l'au moins un point auxiliaire réel ;
• la fourniture de données de point principal absolues qui décrivent la position d'un ou de deux points principaux réels du pelvis par rapport au dispositif marqueur attaché au pelvis, lesdits un ou deux points principaux réels se trouvant dans le plan médio-sagittal et représentant un repère anatomique sur le pelvis ;
• le calcul d'une position du plan médio-sagittal par rapport au dispositif marqueur, dans lequel le calcul utilise les données de point relatives et données de point auxiliaire ainsi que les données de point principal absolues fournies ;
dans lequel,
a) si les données de point principal absolues décrivent la position de deux points principaux réels,
i) alors les données de point relatives décrivent au moins une limite et l'étape de calcul utilise l'au moins une limite ainsi que la position d'au moins un point auxiliaire réel et les positions des deux points principaux réels ; et
ii) alors l'étape du calcul comprend :
la détermination d'au moins un point auxiliaire virtuel en utilisant les données de point relatives, les données de point principal absolues, et les données de point auxiliaire absolues, les données de point relatives limitant les positions possibles en décrivant une distance la plus courte entre le plan médio-sagittal et un point auxiliaire particulier de l'au moins un point auxiliaire réel et/ou en décrivant une distance entre le point auxiliaire virtuel et le point auxiliaire particulier, le point auxiliaire virtuel étant symétrique au point auxiliaire particulier en ce qui concerne le plan médio-sagittal et représentant un repère anatomique sur le pelvis ; ou
b) si les données de point principal absolues décrivent la position d'un point principal réel,
i) alors les données de point relatives décrivent au moins deux limites et l'étape du calcul utilise les au moins deux limites ainsi que les positions d'au moins deux des points auxiliaires réels et la position de l'un point principal réel, les deux points auxiliaires réels étant sur le même côté du plan médio-sagittal, les données de point relatives contenant la position possible du plan médio-sagittal par rapport aux points auxiliaires réels en décrivant une relation positionnelle définie par des distances les plus courtes entre les points auxiliaires réels et le plan médio-sagittal, et
ii) alors l'étape du calcul comprend :
la détermination d'une position d'un point principal virtuel sur la base des données de point relatives, en utilisant deux des au moins deux points auxiliaires réels, les deux points auxiliaires réels définissant une ligne qui croise le plan médio-sagittal ; et
la détermination, sur la base de la position du point principal virtuel et de l'un point principal réel, d'au moins un point auxiliaire virtuel en utilisant les données de point relatives qui décrivent une distance entre le plan médio-sagittal et un point auxiliaire particulier des au moins deux points auxiliaires réels et/ou entre le point auxiliaire virtuel et le point auxiliaire particulier, le point auxiliaire virtuel étant symétrique au point auxiliaire particulier en ce qui concerne le plan médio-sagittal.
- dans lequel la position du plan médio-sagittal est déterminée sur la base du point auxiliaire particulier et du point auxiliaire virtuel symétrique en ce que le plan médio-sagittal est défini pour être perpendiculaire à une ligne reliant le point auxiliaire particulier et le point auxiliaire virtuel symétrique.

2. Procédé de traitement de données selon l'une quelconque des revendications précédentes, dans lequel la position du point auxiliaire virtuel est symétrique à la position du point auxiliaire particulier par rapport au plan médio-sagittal.

3. Procédé de traitement de données selon l'une quelconque des revendications précédentes, comprenant en outre les étapes de :
• la fourniture de données appelées données de pelvis qui limitent les positions relatives possibles entre des repères du pelvis et/ou entre les repères et le plan médio-sagittal ;
• la fourniture de données appelées données de repère qui respectivement corrèlent au moins certains des points principaux réels et/ou virtuels et/ou points auxiliaires avec au moins certains des repères du pelvis ; et
• si plus d'une solution pour calculer la position du plan médio-sagittal est possible, la sélection d'une des solutions possibles pour la position du plan médio-sagittal sur la base des données de repère et données de pelvis.

4. Procédé de traitement de données qui inclut le procédé de traitement de données de l'une quelconque des revendications précédentes et qui est un procédé pour déterminer la position du plan médio-sagittal du pelvis et d'un plan central d'articulation spinale du pelvis, le plan central d'articulation spinale étant appelé SJCP, dans lequel, afin de déterminer le plan médio-sagittal, le procédé de traitement de données de l'une quelconque des revendications précédentes est réalisé, et, afin de déterminer le SJCP, des données de point auxiliaire absolues décrivent la position d'au moins deux des points auxiliaires du pelvis par rapport au dispositif marqueur, les au moins deux points auxiliaires étant à l'extérieur du plan médio-sagittal ; comprenant en outre l'étape de la fourniture de données de plan auxiliaire relatives qui décrivent une relation positionnelle prédéterminée entre le SJCP et le plan médio-sagittal ;
dans lequel la position du SJCP est déterminée en supposant que les au moins deux points auxiliaires se trouvent dans le SJCP, et est déterminée sur la base des données de plan auxiliaire relatives.

5. Procédé de traitement de données qui inclut le procédé de traitement de données de la revendication précédente pour déterminer la position du plan médio-sagittal et du SJCP et qui est en outre un procédé pour déterminer la position d'un plan pelvien antérieur, comprenant en outre les étapes de :
la fourniture de données de plan standard relatives qui décrivent la relation positionnelle prévue entre le SJCP et le plan pelvien antérieur ; et
la détermination de la position du plan pelvien antérieur sur la base de la position du SJCP et des données de plan standard relatives.

6. Procédé de traitement de données selon l'une quelconque des revendications précédentes, dans lequel : un des repères représentés par un des points auxiliaires est le repère de l'épine iliaque antérosupérieure gauche ou droit ; et/ou
les données de plan standard relatives décrivent l'angle entre le plan pelvien antérieur et le SJCP ; et/ou
le plan pelvien antérieur et le SJCP s'intersectent le long d'une ligne reliant les repères de l'épine iliaque antérosupérieure gauche et droit du pelvis ; et/ou
un autre point auxiliaire représente un repère défini par l'acétabulum ou une partie de l'acétabulum ou la fosse de l'acétabulum ou un autre point de l'acétabulum ; et/ou
les données de point relatives décrivent une limite pour une distance inter-fosse ou une distance inter-U du pelvis.

7. Procédé de traitement de données pour déterminer points supplémentaires appelées points supplémentaires auxiliaires qui peuvent être utilisés pour déterminer la position du plan médio-sagittal, selon l'une quelconque des revendications précédentes, comprenant les étapes de :
la fourniture d'au moins un point auxiliaire ;
la fourniture de données de point auxiliaire relatives qui décrivent au moins une limite qui limite les positions possibles pour un point auxiliaire supplémentaire, le point auxiliaire supplémentaire étant symétrique à un des un ou plusieurs points auxiliaires par rapport au plan médio-sagittal ; et
la fourniture de données de point candidat par étapes, dans lequel, dans chaque étape, la position d'un nouveau point candidat est décrite par les données de point candidat, dans lequel, dans l'étape actuelle, les étapes suivantes sont réalisées :
a) la fourniture de nouvelles données appelées données de nouveau point candidat qui décrivent une position relative d'un nouveau point candidat par rapport au dispositif marqueur, le nouveau point candidat étant un nouveau candidate pour une position du point auxiliaire supplémentaire ;
b) le contrôle que les positions du nouveau point candidat respectent ou non l'au moins une limite décrite par les données de point auxiliaire relatives ;
c) la répétition des étapes a) et b) si la position du nouveau point candidat ne respecte pas l'au moins une limite, ou l'acceptation du nouveau point candidat en tant que point auxiliaire supplémentaire si la position du point candidat respecte l'au moins une limite.

8. Programme, qui, lorsqu'il est exécuté sur un ordinateur ou est chargé sur un ordinateur, fait en sorte que l'ordinateur réalise le procédé selon l'une quelconque des revendications précédentes.

9. Support de stockage de programme, sur lequel le programme de la revendication 8 est stocké.

10. Ordinateur, sur lequel le programme de la revendication 8 est exécuté ou dans la mémoire duquel le programme de la revendication 7 est chargé.

11. Système de navigation pour chirurgie assistée par ordinateur, comprenant :
l'ordinateur de la revendication 10 ;
un dispositif de détection pour détecter la position des points principal et auxiliaire et pour générer des signaux de détection qui représentent la position des points principal et auxiliaire et pour fournir les signaux de détection à l'ordinateur de la revendication 9, l'ordinateur étant conçu pour traiter les données de point principal absolues et les données de point auxiliaire absolues, et les données de point relatives conformément à l'une quelconque des revendications de procédé 1 à 6 ; et
une interface utilisateur pour recevoir des données à partir de l'ordinateur afin de fournir des informations à l'utilisateur, l'ordinateur étant conçu pour calculer les données conformément au procédé de traitement de données selon l'une quelconque des revendications de procédé précédentes.

12. Procédé de détermination pour déterminer la position d'un plan médio-sagittal d'un pelvis, comprenant les étapes de :
la génération de signaux de détection en détectant un pointeur, ledit pointeur entrant en contact avec un pelvis ou un outil, appelé outil de détection de point principal ;
la détermination, sur la base des signaux de détection, des données de point principal absolues et données de point auxiliaire absolues selon l'une quelconque des revendications de procédé 1 à 6, et puis la réalisation de l'une quelconque des revendications de procédé 1 à 6 sur la base des données de point principal absolues et données de points auxiliaires absolues déterminées.

13. Système de navigation selon la revendication 11, comprenant en outre un outil, appelé outil de détection de point de plan, pour détecter au moins un point principal réel, l'outil de détection de point de plan comprenant :
un côté proximal constitué pour être ajusté sur un patient en entrant en contact avec le patient le long d'une première direction d'extension en longueur de l'outil, le côté proximal étant constitué pour être capable d'être aligné avec un plan d'un pelvis le long de la première direction d'extension en longueur, la direction dans laquelle le côté proximal est tourné et le long de laquelle l'outil peut être fléchi pour mettre l'outil en contact avec le patient étant une troisième direction d'extension de l'outil ;
un côté distal qui comprend au moins un élément d'insertion de pointeur comprenant un évidement effilé pour insérer l'embout d'un pointeur, l'au moins un élément d'insertion de pointeur étant positionné de façon saillante dans une première section du côté distal qui est, dans la direction d'extension en longueur, plus près d'une première extrémité de l'outil de détection de point de plan que d'une seconde extrémité ;
dans lequel le côté distal comprend en outre une seconde section ayant une surface plate et étant plus près de la seconde extrémité que de la première extrémité ;
le côté proximal et le côté distal s'étendant tous les deux dans la première direction et dans une deuxième direction d'extension, la deuxième direction étant transversale à la première direction,
l'outil pouvant être fléchi dans la troisième direction tout en étant rigide dans la deuxième direction, la deuxième direction étant une direction d'extension en largeur de l'outil, l'extension en largeur de l'outil étant plus petite que l'extension en longueur.
